(19) Europäisches Patentamt
European Patent Office
Office européen des brevets

(11) Veröffentlichungsnummer : **0 566 534 A1**

(12) **EUROPÄISCHE PATENTANMELDUNG**

(21) Anmeldenummer : **93810245.6**

(22) Anmeldetag : **06.04.93**

(51) Int. Cl.$^5$ : **C07C 309/65,** C07C 281/14, C07C 323/48, C07C 337/08, A01N 33/26, A01N 41/04, A01N 47/34, A01N 47/42

(30) Priorität : **16.04.92 CH 1267/92**

(43) Veröffentlichungstag der Anmeldung : **20.10.93 Patentblatt 93/42**

(84) Benannte Vertragsstaaten : **AT BE CH DE DK ES FR GB GR IE IT LI LU MC NL PT SE**

(71) Anmelder : **CIBA-GEIGY AG**
**Klybeckstrasse 141**
**CH-4002 Basel (CH)**

(72) Erfinder : **Dürr, Dieter, Dr.**
**Brändelistalweg 16**
**CH-4103 Bottmingen (CH)**
Erfinder : **Hall, Roger Graham, Dr.**
**Hauptstrasse 5**
**CH-4147 Aesch (CH)**
Erfinder : **Ehrenfreund, Josef, Dr.**
**Amselstrasse 11**
**CH-4123 Allschwil (CH)**
Erfinder : **Pascual, Alfons, Dr.**
**Gundeldingerstrasse 433**
**CH-4053 Basel (CH)**

(54) **Benzophenonderivate.**

(57) Verbindungen der Formel

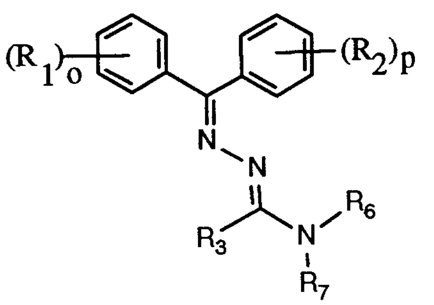

(I),

worin
o und p unabhängig voneinander 0, 1, 2, 3, 4 oder 5, wobei, wenn o grösser als 1 ist, die Reste $R_1$ gleich oder verschieden sind und wobei, wenn p grösser als 1 ist, die Reste $R_2$ gleich oder verschieden sind ;
$R_1$ und $R_2$ unabhängig voneinander $C_1$-$C_4$-Alkyl, Halogen-$C_1$-$C_4$-alkyl, $C_1$-$C_4$-Alkoxy, Halogen-$C_1$-$C_4$-alkoxy, $C_1$-$C_4$-Alkylthio, Halogen-$C_1$-$C_4$-alkylthio, Halogen, -OH, Nitro, Cyano, Phenoxy, -O-S(=O)-$R_8$ oder -O-S(=O)$_2$-$R_8$, $C_2$-$C_4$-Alkinyl, durch Phenyl oder Halogen substituiertes $C_2$-$C_4$-Alkinyl, $C_2$-$C_4$-Alkenyl, Halogen-$C_2$-$C_4$-alkenyl, und/oder zwei an benachbarte C-Atome des Phenylrings gebundene Substituenten $R_1$ und/oder zwei an benachbarte C-Atome des Phenylrings gebundene Substituenten $R_2$ unabhängig voneinander gemeinsam -$Y_1$-Z-$Y_2$- sind ;
$R_3$ Wasserstoff, Halogen, -OH, -SH, $C_1$-$C_4$-Alkyl, $C_3$-$C_6$-Cycloalkyl, Halogen-$C_1$-$C_4$-alkyl, $C_1$-$C_4$-Alkoxy, $C_1$-$C_4$-Alkoxy-$C_1$-$C_4$-alkyl, $C_1$-$C_4$-Alkoxy-$C_1$-$C_4$-alkoxy, $C_1$-$C_4$-Alkylthio oder -N($R_4$)$R_5$ ist ;
$R_4$ und $R_8$ unabhängig voneinander Wasserstoff, $C_1$-$C_4$-Alkyl oder -OH sind ;
$R_6$ -O$R_9$, -S(O)$_n$$R_{10}$, -P(O)$R_{11}$$R_{12}$ oder -N$R_{13}$$R_{14}$ ;
$R_7$ Wasserstoff, $C_1$-$C_4$-Alkyl, $C_3$-$C_6$-Cycloalkyl, Halogen-$C_1$-$C_4$-alkyl, $C_1$-$C_4$-Alkoxy-$C_1$-$C_4$-alkyl, Allyl, $C_1$-$C_3$-Alkylallyl, Halogenallyl oder Propargyl ;
$R_6$ $C_1$-$C_8$-Alkyl oder Halogen-$C_1$-$C_8$-alkyl ;
$R_9$ Wasserstoff, $C_1$-$C_4$-Alkyl, Halogen-$C_1$-$C_4$-alkyl, $C_1$-$C_4$-Alkoxy-$C_1$-$C_4$-alkyl, $C_3$-$C_6$-Cycloalkyl, $C_2$-$C_6$-Alkenyl, Halogen-$C_2$-$C_6$-alkenyl oder Propargyl ;
$R_{10}$ Wasserstoff, $C_1$-$C_4$-Alkyl, Halogen-$C_1$-$C_4$-alkyl, Phenyl oder -N$R_{13}$$R_{14}$ ist ;
$R_{11}$ und $R_{12}$ unabhängig voneinander $C_1$-$C_4$-Alkoxy sind ;
$R_{13}$ und $R_{14}$ unabhängig voneinander Wasserstoff, $C_1$-$C_4$-Alkyl, Halogen-$C_1$-$C_4$-alkyl, Phenyl, $C_1$-$C_4$-Alkylamido, $C_1$-$C_4$-Dialkylamido oder -$NH_2$ sind ;

EP 0 566 534 A1

n 0, 1 oder 2 ;
$Y_1$ und $Y_2$ unabhängig voneinander O oder S sind ; und
Z Methylen, Eth-1,2-ylen ; Halogenmethylen oder Halogeneth-1,2-ylen bedeutet, und gegebenenfalls Tautomere davon sowie deren Salze können als agrochemische Wirkstoffe verwendet werden und sind in an sich bekannter Weise herstellbar.

Die Erfindung betrifft Verbindungen der Formel

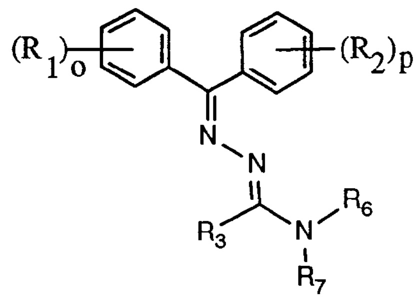

(I),

worin

o und p unabhängig voneinander 0, 1, 2, 3, 4 oder 5, wobei, wenn o grösser als 1 ist, die Reste $R_1$ gleich oder verschieden sind und wobei, wenn p grösser als 1 ist, die Reste $R_2$ gleich oder verschieden sind;

$R_1$ und $R_2$ unabhängig voneinander $C_1$-$C_4$-Alkyl, Halogen-$C_1$-$C_4$-alkyl, $C_1$-$C_4$-Alkoxy, Halogen-$C_1$-$C_4$-alkoxy, $C_1$-$C_4$-Alkylthio, Halogen-$C_1$-$C_4$-alkylthio, Halogen, -OH, Nitro, Cyano, Phenoxy, -O-S(=O)-$R_8$ oder -O-S(=O)$_2$-$R_8$, $C_2$-$C_4$-Alkinyl, durch Phenyl oder Halogen substituiertes $C_2$-$C_4$-Alkinyl, $C_2$-$C_4$-Alkenyl, Halogen-$C_2$-$C_4$-alkenyl, und/oder zwei an benachbarte C-Atome des Phenylrings gebundene Substituenten $R_1$ und/oder zwei an benachbarte C-Atome des Phenylrings gebundene Substituenten $R_2$ unabhängig voneinander gemeinsam -$Y_1$-Z-$Y_2$- sind;

$R_3$ Wasserstoff, Halogen, -OH, -SH, $C_1$-$C_4$-Alkyl, $C_3$-$C_6$-Cycloalkyl, Halogen-$C_1$-$C_4$-alkyl, $C_1$-$C_4$-Alkoxy, $C_1$-$C_4$-Alkoxy-$C_1$-$C_4$-alkyl, $C_1$-$C_4$-Alkoxy-$C_1$-$C_4$-alkoxy, $C_1$-$C_4$-Alkylthio oder -N($R_4$)$R_5$ ist;

$R_4$ und $R_8$ unabhängig voneinander Wasserstoff, $C_1$-$C_4$-Alkyl oder -OH sind;

$R_8$ -O$R_9$, -S(O)$_n$$R_{10}$, -P(O)$R_{11}$$R_{12}$ oder -N$R_{13}$$R_{14}$;

$R_7$ Wasserstoff, $C_1$-$C_4$-Alkyl, $C_3$-$C_6$-Cycloalkyl, Halogen-$C_1$-$C_4$-alkyl, $C_1$-$C_4$-Alkoxy-$C_1$-$C_4$-Alkyl, Allyl, $C_1$-$C_3$-Alkylallyl, Halogenallyl oder Propargyl;

$R_8$ $C_1$-$C_8$-Alkyl oder Halogen-$C_1$-$C_8$-alkyl;

$R_9$ Wasserstoff, $C_1$-$C_4$-Alkyl, Halogen-$C_1$-$C_4$-alkyl, $C_1$-$C_4$-Alkoxy-$C_1$-$C_4$-alkyl, $C_3$-$C_6$-Cycloalkyl, $C_2$-$C_6$-Alkenyl, Halogen-$C_2$-$C_6$-alkenyl oder Propargyl;

$R_{10}$ Wasserstoff, $C_1$-$C_4$-Alkyl, Halogen-$C_1$-$C_4$-alkyl, Phenyl oder -N$R_{13}$$R_{14}$ ist;

$R_{11}$ und $R_{12}$ unabhängig voneinander $C_1$-$C_4$-Alkoxy sind;

$R_{13}$ und $R_{14}$ unabhängig voneinander Wasserstoff, $C_1$-$C_4$-Alkyl, Halogen-$C_1$-$C_4$-alkyl, Phenyl, $C_1$-$C_4$-Alkylamido, $C_1$-$C_4$-Dialkylamido oder -$NH_2$ sind;

n 0, 1 oder 2 ist;

$Y_1$ und $Y_2$ unabhängig voneinander O oder S sind; und

Z Methylen, Eth-1,2-ylen; Halogenmethylen oder Halogeneth-1,2-ylen bedeutet,

Tautomere der Verbindungen der Formel I, sowie Salze der verschiedenen Tautomeren, ein Verfahren zur Herstellung und die Verwendung dieser Verbindungen und Tautomere, Schädlingsbekämpfungsmittel enthaltend eine Verbindung der Formel I oder eines Tautomeren oder eines agrochemisch verwendbaren Salzes davon, und ein Verfahren zur Herstellung der Mittel und die Verwendung dieser Mittel.

Die Verbindungen der Formel I können teilweise als Tautomere vorliegen. Bedeutet z.B. der Rest $R_3$ Hydroxy oder der Rest $R_7$ Wasserstoff, können die entsprechenden Verbindungen der Fomel I als Gleichgewichtsgemisch von Tautomeren vorliegen. Demgemäss sind unter den Verbindungen der Formel I auch entsprechende Tautomere oder deren Salze zu verstehen, auch wenn sie nicht in jedem Fall speziell erwähnt werden.

Die Verbindungen der Formel I und gegebenenfalls ihre Tautomeren können als Salze vorliegen. Verbindungen der Formel I mit mindestens einem basischen Zentrum können z. B. Säureadditionssalze bilden. Diese werden beispielsweise mit starken anorganischen Säuren, wie Mineralsäuren, z. B. Schwefelsäure, einer Phosphorsäure oder einer Halogenwasserstoffsäure, mit starken organischen Carbonsäuren, wie gegebenenfalls, z. B. durch Halogen, substituierten $C_1$-$C_4$-Alkancarbonsäuren, z. B. Essigsäure, wie gegebenenfalls ungesättigten Dicarbonsäuren, z. B. Oxal-, Malon-, Malein-, Fumar- oder Phthalsäure, wie Hydroxycarbonsäuren, z. B. Ascorbin-, Milch-, Äpfel-, Wein- oder Zitronensäure, oder wie Benzoesäure, oder mit organischen Sulfonsäuren, wie gegebenenfalls, z. B. durch Halogen, substituierten $C_1$-$C_4$-Alkan- oder Aryl-sulfonsäuren, z. B. Methan- oder p-Toluolsulfonsäure, gebildet. Verbindungen der Formel I mit mindestens einer aciden Gruppe können Salze mit Basen bilden. Geeignete Salze mit Basen sind beispielsweise Metallsalze, wie Alkali- oder Erdalkalimetallsalze, z.B. Natrium-, Kalium- oder Magnesiumsalze, oder Salze mit Ammoniak oder einem organischen Amin, wie Morpholin, Piperidin, Pyrrolidin, einem Mono-, Di- oder Triniederalkylamin, z. B. Ethyl-, Diethyl-, Triethyl- oder Dimethyl-propyl-amin, oder einem Mono-, Di- oder Trihydroxyniederalkylamin, z. B. Mono-, Di- oder Triethanolamin. Weiterhin können gegebenenfalls entsprechende innere Salze gebildet werden. Bevorzugt sind im Rahmen der Erfindung agrochemisch vorteilhafte Salze; umfasst sind aber auch für agrochemische Verwendungen mit Nachteilen behaftete, z. B. bienen- oder fisch-toxische, Salze, die beispielsweise für die Isolierung bzw. Reinigung von freien Verbindungen der Formel I oder deren agrochemisch verwendbaren Salzen eingesetzt werden. Der Begriff "Verbindung der Formel I" umfasst also auch die Tautomeren dieser Verbindungen, ihre Salze sowie die Salze der Tautomeren.

Halogen - per se oder als Strukturelement von Gruppen und Verbindungen, wie von Halogenalkyl und Halogenallyl, - ist Fluor, Chlor, Brom oder Iod, insbesondere Fluor, Chlor oder Brom, vor allem Fluor oder Chlor.

Kohlenstoffhaltige Gruppen und Verbindungen enthalten, sofern nicht abweichend definiert, jeweils 1 bis und mit 8, vorzugsweise 1 bis 4, insbesondere 1 oder 2, Kohlenstoffatome.

$C_3$-$C_6$-Cycloalkyl ist Cyclopropyl, Cyclobutyl, Cyclopentyl oder Cyclohexyl.

Alkyl - als Gruppe per se sowie als Strukturelement von anderen Gruppen und Verbindungen, wie von Halogenalkyl, Alkoxy, Alkoxyalkyl, Alkylthio und Alkylallyl, - ist, jeweils unter gebührender Berücksichtigung der von Fall zu Fall umfassten Anzahl der in der entsprechenden Gruppe oder Verbindung enthaltenen Kohlenstoffatome, entweder geradkettig, d. h. Methyl, Ethyl, Propyl oder Butyl, oder verzweigt, d. h. Isopropyl, Isobutyl, sek.-Butyl oder tert.-Butyl, Pentyl, Hexyl, Heptyl oder Octyl oder deren Isomere.

Halogenalkyl, Halogenalkenyl und Halogenallyl können teilweise halogeniert oder perhalogeniert sein. Beispiele für Halogenalkyl sind das ein- bis dreifach durch Fluor, Chlor und/oder Brom substituierte Methyl, wie $CHF_2$, $CHCl_2$, $CH_2Cl$ oder $CF_3$; das ein- bis fünffach durch Fluor, Chlor und/oder Brom substituierte Ethyl, wie $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ oder CClFCHClF; das ein- bis siebenfach durch Fluor, Chlor und/oder Brom substituierte Propyl oder Isopropyl, wie $CH_2CHBrCH_2Br$, $CF_2CHFCF_3$, $CH_2CF_2CF_3$ oder $CH(CF_3)_2$; und das ein- bis neunfach durch Fluor, Chlor und/oder Brom substituierte Butyl oder eines seiner Isomeren, wie $CF(CF_3)CHFCF_3$ oder $CH_2(CF_2)_2CF_3$. Beispiele für Halogenallyl sind 3-Chlorprop-2-en-1-yl, 3,3-Difluorprop-2-en-1-yl und 1,1,2,3,3-Pentafluorprop-2-en-1-yl.

$C_1$-$C_4$-Alkoxy-$C_1$-$C_4$-alkyl und $C_1$-$C_4$-Alkoxy-$C_1$-$C_4$-alkoxy sind Alkyl- bzw. Alkoxygruppen, die einfach durch Alkoxy substituiert sind, wobei beide Kohlenstoffketten unabhängig voneinander gerade oder verzweigt sein können; Beispiele sind Methoxymethyl, 2-Methoxyethyl, Ethoxymethyl, 2-Isopropoxyethyl, 2-Propoxypropyl oder 4-Methoxybut-2-yl, 2-Methoxyethoxy, Ethoxymethoxy oder 2-Isopropoxyethoxy.

$C_2$-$C_6$-Alkenylgruppen sind geradkettige oder verzweigte Alkengruppen, also beispielsweise Hex-3-en-1-yl, Pent-4-en-1-yl oder 2-Methyl-but-3-en-1-yl.

$C_1$-$C_3$-Alkylallylgruppen sind Allylgruppen, die einfach durch geradkettiges oder verzweigtes Alkyl substituiert sind, beispielsweise But-2-en-1-yl, Pent-2-en-1-yl, 2-Methylallyl oder 4-Methyl-pent-2-en-1-yl.

Bevorzugte Ausführungsformen im Rahmen der Erfindung sind

1) Eine Verbindung der Formel I, worin

$R_1$ in 4-Stellung gebundenes -O-S(=O)$_2$-$C_1$-$C_4$-Alkyl oder -O-S(=O)$_2$-Halogen-$C_1$-$C_4$-alkyl;

$R_2$ in 4-Stellung gebundenes Halogen oder $CF_3$;

$R_3$ Wasserstoff, Halogen, -OH, -SH, $C_1$-$C_4$-Alkyl, $C_3$-$C_6$-Cycloalkyl, Halogen-$C_1$-$C_4$-alkyl, $C_1$-$C_4$-Alkoxy, $C_1$-$C_4$-Alkylthio oder -N($R_4$)$R_5$;

$R_4$ und $R_5$ unabhängig voneinander Wasserstoff oder $C_1$-$C_4$-Alkyl;

$R_6$ O$R_9$;

$R_7$ Wasserstoff, $C_1$-$C_4$-Alkyl, Halogen-$C_1$-$C_4$-alkyl, $C_1$-$C_4$-Alkoxy-$C_1$-$C_4$-alkyl, Allyl, Halogenallyl, $C_1$-$C_3$-Alkylallyl oder Propargyl;

$R_9$ Wasserstoff, $C_1$-$C_4$-Alkyl, Halogen-$C_1$-$C_4$-alkyl, $C_1$-$C_4$-Alkoxy-$C_1$-$C_4$-alkyl, Allyl, $C_1$-$C_3$-Alkylallyl oder Propargyl; und

o und p 1 bedeutet, und gegebenenfalls Tautomere davon;

2) Eine Verbindung der Formel I, worin

die Reste $R_1$ unabhängig voneinander $C_1$-$C_4$-Alkyl, Halogen-$C_1$-$C_4$-alkyl, $C_1$-$C_4$-Alkoxy, Halogen-$C_1$-$C_4$-alkoxy, Halogen, -O-S(=O)$_2$-$C_1$-$C_4$-Alkyl oder -O-S(=O)$_2$-Halogen-$C_1$-$C_4$-alkyl, Phenoxy oder zwei Reste $R_1$ gemeinsam -O$CF_2$-O-;

besonders Halogen-$C_1$-$C_4$-alkyl, Halogen-$C_1$-$C_4$-alkoxy, Halogen, -O-S(=O)$_2$-$C_1$-$C_2$-Alkyl oder -O-S(=O)$_2$-Halogen-$C_1$-$C_2$-alkyl;

ganz besonders -O-S(=O)$_2$-$C_1$-$C_2$-Alkyl, -O-S(=O)$_2$-Halogen-$C_1$-$C_2$-alkyl oder $C_1$-$C_3$-Fluoralkoxy;

besonders bevorzugt -O-S(=O)$_2$-$CH_3$, -O-S(=O)$_2$-$CF_3$ oder -O$CF_2CHFCF_3$, und

o 1 oder 2,

insbesondere $R_1$ in 3- oder 4-Stellung gebundenes $-O-S(=O)_2-CF_3$ und o 1 bedeutet, oder gegebenenfalls Tautomere davon;

3) Eine Verbindung der Formel I, worin

die Reste $R_2$ unabhängig voneinander $C_1$-$C_4$-Alkyl, Halogen-$C_1$-$C_4$-alkyl, $C_1$-$C_4$-Alkoxy, Halogen-$C_1$-$C_4$-alkoxy, Halogen, $-O-S(=O)_2-C_1-C_2$-Alkyl oder $-O-S(=O)_2$-Halogen-$C_1$-$C_2$-alkyl;

besonders Fluor, Chlor, Brom oder $-CF_3$; und

p 1 oder 2;

ganz besonders worin $R_2$ in 4-Stellung gebundenes Chlor und p 1 bedeutet; oder gegebenenfalls Tautomere davon;

4) Eine Verbindung der Formel I, worin

$R_3$ Wasserstoff, Halogen, -OH, -SH, $C_1$-$C_4$-Alkyl, $C_3$-$C_6$-Cycloalkyl, Halogen-$C_1$-$C_4$-alkyl, $C_1$-$C_4$-Alkoxy, $C_1$-$C_4$-Alkoxy-$C_1$-$C_4$-alkoxy, $C_1$-$C_4$-Alkylthio, $-NH_2$, $-NH(C_1-C_2$-Alkyl), $-N(C_1-C_2$-Alkyl$)_2$ oder -NHOH;

besonders Wasserstoff, -OH, -SH, $C_1$-$C_2$-Alkyl, Cyclopropyl, $C_1$-$C_2$-Alkoxy, $C_1$-$C_2$-Alkoxy-$C_1$-$C_2$-alkoxy, $C_1$-$C_2$-Alkylthio, $-NH_2$, $-NH(CH_3)$, $-N(CH_3)_2$ oder -NHOH;

besonders bevorzugt Wasserstoff, -OH, -SH, Methyl, Cyclopropyl, Methoxy, Methylthio, $-NH_2$, $-NH(CH_3)$, $-N(CH_3)_2$ oder -NHOH bedeutet; oder gegebenenfalls Tautomere davon;

5) Eine Verbindung der Formel I, worin

$R_6$ $-OR_9$ und $R_9$ Wasserstoff, $C_1$-$C_4$-Alkyl, $C_2$-$C_6$-Alkenyl oder Halogen-$C_2$-$C_6$-alkenyl;

insbesondere $R_9$ Wasserstoff, $C_1$-$C_2$-Alkyl, $C_2$-$C_3$-Alkenyl oder Halogen-$C_2$-$C_3$-alkenyl;

ganz besonders worin $R_9$ Methyl oder Ethyl bedeutet, oder gegebenenfalls Tautomere davon;

6) Eine Verbindung der Formel I, worin

$R_6$ $-SO_2-C_1-C_4$-Alkyl, $-SO_2NH(CH_3)$, $-P(O)(OC_2H_5)_2$, $-NH_2$, $-N(CH_3)NH_2$ oder $-NHCON(CH_3)_2$ bedeutet, oder gegebenenfalls Tautomere davon;

7) Eine Verbindung der Formel I, worin

$R_7$ Wasserstoff, $C_1$-$C_2$-Alkyl, $C_1$-$C_2$-Alkoxy-$C_1$-$C_2$-alkyl, Allyl, 2-Methylallyl, Halogenallyl oder Propargyl; insbesondere Wasserstoff, Methyl oder Ethyl, ganz besonders Methyl bedeutet, oder gegebenenfalls Tautomere davon;

8) Eine Verbindung der Formel I, worin

$R_1$ $-O-S(=O)_2-C_1-C_2$-Alkyl, $-O-S(=O)_2$-Halogen-$C_1$-$C_2$-alkyl oder $C_1$-$C_3$-Fluoralkoxy;

$R_2$ Fluor oder Chlor;

$R_3$ Wasserstoff, -OH, -SH, $C_1$-$C_2$-Alkyl, Cyclopropyl, $C_1$-$C_2$-Alkoxy, $C_1$-$C_2$-Alkoxy-$C_1$-$C_2$-alkoxy, $C_1$-$C_2$-Alkylthio, $-NH_2$, $-NH(CH_3)$, $-N(CH_3)_2$ oder -NHOH;

$R_6$ $-OR_9$, $-SO_2-C_1-C_4$-Alkyl, $-SO_2NH(CH_3)$, $-P(O)(OC_2H_5)_2$, $-NH_2$, $-N(CH_3)NH_2$ oder $-NHCON(CH_3)_2$;

$R_7$ Wasserstoff, Methyl oder Ethyl; und

$R_9$ Wasserstoff, $C_1$-$C_2$-Alkyl, $C_2$-$C_3$-Alkenyl oder Halogen-$C_2$-$C_3$-alkenyl und

o und p 1 oder 2 bedeuten, oder gegebenenfalls Tautomere davon;

9) Eine Verbindung der Formel I, worin

$R_1$ $-O-S(=O)_2-CH_3$, $-O-S(=O)_2-CF_3$ oder $-OCF_2CHFCF_3$;

$R_2$ Chlor;

$R_3$ Wasserstoff, -OH, -SH, $C_1$-$C_2$-Alkyl, Cyclopropyl, $C_1$-$C_2$-Alkoxy, $C_1$-$C_2$-Alkoxy-$C_1$-$C_2$-alkoxy, $C_1$-$C_2$-Alkylthio, $-NH_2$, $-NH(CH_3)$, $-N(CH_3)_2$ oder -NHOH;

$R_6$ $-OR_9$, $-SO_2-C_1-C_4$-Alkyl, $-SO_2NH(CH_3)$, $-P(O)(OC_2H_5)_2$, $-NH_2$, $-N(CH_3)NH_2$ oder $-NHCON(CH_3)_2$;

$R_7$ Wasserstoff, Methyl oder Ethyl; und

$R_9$ Wasserstoff, $C_1$-$C_2$-Alkyl, $C_2$-$C_3$-Alkenyl oder Halogen-$C_2$-$C_3$-alkenyl und

o und p 1 oder 2 bedeuten, oder gegebenenfalls Tautomere davon; sowie

10) Eine Verbindung der Formel I, worin

$R_1$ in 4-Stellung gebundenes $-O-S(=O)_2-CF_3$;

$R_2$ in 4-Stellung gebundenes Chlor;

$R_3$ Wasserstoff, -OH, -SH, $C_1$-$C_2$-Alkyl, Cyclopropyl, $C_1$-$C_2$-Alkoxy, $C_1$-$C_2$-Alkoxy-$C_1$-$C_2$-alkoxy, $C_1$-$C_2$-Alkylthio, $-NH_2$, $-NH(CH_3)$, $-N(CH_3)_2$ oder -NHOH;

$R_6$ $-OR_9$, $-SO_2-C_1-C_4$-Alkyl, $-SO_2NH(CH_3)$, $-P(O)(OC_2H_5)_2$, $-NH_2$, $-N(CH_3)NH_2$ oder $-NHCON(CH_3)_2$;

$R_7$ Wasserstoff, Methyl oder Ethyl; und

$R_9$ Wasserstoff, $C_1$-$C_2$-Alkyl, $C_2$-$C_3$-Alkenyl oder Halogen-$C_2$-$C_3$-alkenyl und

o und p 1 bedeuten, oder gegebenenfalls Tautomere davon.

Besonders bevorzugt sind im Rahmen der Erfindung die in den Beispielen H1 bis H6 genannten Verbindungen der Formel I und gegebenenfalls deren Tautomere.

Namentlich bevorzugt sind im Rahmen der Erfindung

(a) 1-(4-Chlorphenyl)-4-hydroxy-5-methyl-6-oxa-2,3,5-triaza-1-(4-trifluormethansulfonyloxyphenyl)-hepta-1,3-dien,

bzw. 1-(4-Chlorphenyl)-5-methyl-6-oxa-4-oxo-2,3,5-triaza-1-(4-trifluormethansulfonyloxyphenyl)-hept-1-en;

(b) 1-(4-Chlorphenyl)-4,5-dimethyl-6-oxa-2,3,5-triaza-1-(4-trifluormethansulfonyloxyphenyl)-hepta-1,3-dien;

(c) 1-(4-Chlorphenyl)-4-hydroxy-5-methyl-6-oxa-2,3,5-triaza-1-(4-methansulfonyloxyphenyl)-hepta-1,3-dien,

bzw. 1-(4-Chlorphenyl)-5-methyl-6-oxa-4-oxo-2,3,5-triaza-1-(4-methansulfonyloxyphenyl)-hept-1-en;

(d) 1-(4-Fluorphenyl)-4-hydroxy-5-methyl-6-oxa-2,3,5-triaza-1-(4-trifluormethansulfonyloxyphenyl)-hepta-1,3-dien,

bzw. 1-(4-Fluorphenyl)-5-methyl-6-oxa-4-oxo-2,3,5-triaza-1-(4-trifluormethansulfonyloxyphenyl)-hept-1-en;

(e) 1-(4-Chlorphenyl)-4,5-dimethyl-6-oxa-2,3,5-triaza-1-(4-methansulfonyloxyphenyl)-hepta-1,3-dien;

(f) 1-(4-Chlorphenyl)-4-cyclopropyl-5-methyl-6-oxa-2,3,5-triaza-1-(4-trifluormethansulfonyloxyphenyl)-hepta-1,3-dien;

(g) 1-(4-Fluorphenyl)-4,5-dimethyl-6-oxa-2,3,5-triaza-1-(4-trifluormethansulfonyloxyphenyl)-hepta-1,3-dien;

(h) 1-(4-Chlorphenyl)-4-cyclopropyl-5-ethyl-6-oxa-2,3,5-triaza-1-(4-trifluormethansulfonyloxyphenyl)-hepta-1,3-dien;

(i) 1-(4-Chlorphenyl)-4,5-dimethyl-6-oxa-2,3,5-triaza-1-(4-trifluormethansulfonyloxyphenyl)-octa-1,3-dien;

(j) 1-(4-Chlorphenyl)-5-ethyl-4-methyl-6-oxa-2,3,5-triaza-1-(4-trifluormethansulfonyloxyphenyl)-hepta-1,3-dien, und

(k) 1-(4-Chlorphenyl)-4-hydroxy-5-methyl-6-oxa-2,3,5-triaza-1-(4-trifluormethansulfonyloxyphenyl)-nona-1,3,8-trien,

bzw. 1-(4-Chlorphenyl)-5-methyl-6-oxa-4-oxo-2,3,5-triaza-1-(4-trifluormethansulfonyloxyphenyl)-nona-1,8-dien.

Als weiterer Gegenstand der Erfindung ist das Verfahren zur Herstellung der Verbindungen der Formel I und gegebenenfalls ihrer Tautomeren oder eines Salzes davon, beispielsweise dadurch gekennzeichnet, dass man

a) zur Herstellung einer Verbindung der Formel I, worin $R_3$ für -OH, -SH, $-NHR_4$ oder $-NHR_5$ steht, eine Verbindung der Formel

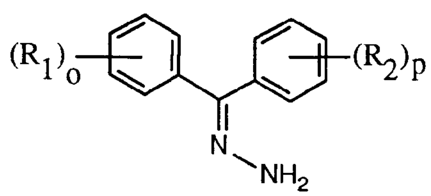

(II)

oder ein Salz davon, vorzugsweise in Gegenwart einer Base, mit einer Verbindung der Formel

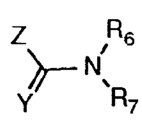

(III),

worin Y für O, S, $-NR_4$ oder $-NR_5$ steht und Z Halogen, $C_1$-$C_8$-Alkoxy, $C_1$-$C_8$-Alkylthio, $C_1$-$C_8$-Alkanoyloxy, $C_1$-$C_8$-Alkansulfonyloxy, Halogen-$C_1$-$C_8$-alkansulfonyloxy, Benzolsulfonyloxy oder Toluolsulfonyloxy bedeutet, oder einem Salz und/oder gegebenenfalls einem Tautomeren davon umsetzt oder

b) eine Verbindung der Formel

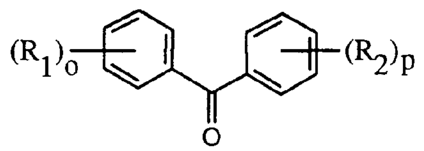

(IV),

vorzugsweise in Gegenwart einer Säure, mit einer Verbindung der Formel

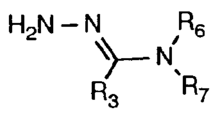

(V)

oder einem Salz und/oder gegebenenfalls einem Tautomeren davon umsetzt oder

c) zur Herstellung einer Verbindung der Formel I, worin $R_3$ -OH, -SH, $C_1$-$C_4$-Alkoxy, $C_1$-$C_4$-Alkylthio oder -$N(R_4)R_5$ ist, eine Verbindung der Formel

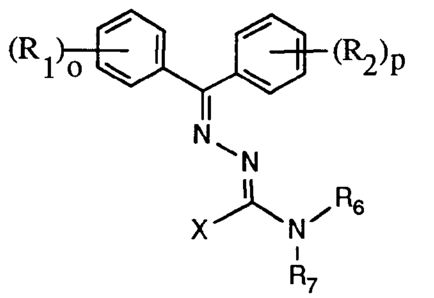

(VI),

worin X Halogen bedeutet, oder ein Salz und/oder gegebenenfalls ein Tautomeres davon, vorzugsweise in Gegenwart einer Base, mit Wasser, $H_2S$, einem $C_1$-$C_4$-Alkanol, einem $C_1$-$C_4$-Alkoxy-$C_1$-$C_4$-alkohol, einem Mercapto-$C_1$-$C_4$-alkan oder einer Verbindung der Formel $HN(R_4)R_5$ oder einem Salz davon umsetzt, oder

d) zur Herstellung einer Verbindung der Formel I, worin $R_3$ Wasserstoff, $C_1$-$C_4$-Alkyl, Halogen-$C_1$-$C_4$-Alkyl oder $C_3$-$C_6$-Cycloalkyl bedeutet, eine Verbindung der Formel

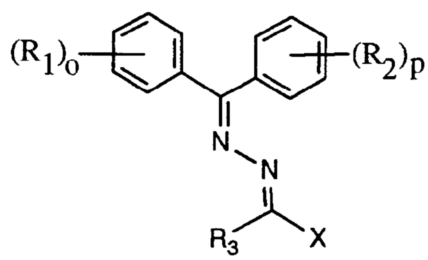

(VII),

worin X Halogen bedeutet, oder ein Salz davon mit einer Verbindung der Formel $HN(R_6)R_7$ oder einem Salz davon, vorzugsweise in Gegenwart einer Base, umsetzt oder

e) zur Herstellung einer Verbindung der Formel I, worin $R_3$ Halogen, vorzugsweise Chlor oder Brom, bedeutet, eine Verbindung der Formel I, worin $R_3$ für -OH oder -SH steht, oder ein Salz und/oder gegebenenfalls ein Tautomeres davon mit einem Halogenierungsmittel, wie $PCl_3$, $PCl_5$, $POCl_3$, $PBr_3$, $SOCl_2$, $SOBr_2$ oder $COCl_2$, vorzugsweise in Gegenwart einer Base, umsetzt

und jeweils, wenn erwünscht, eine verfahrensgemäss oder auf andere Weise erhältliche Verbindung der Formel I oder ein Tautomeres davon, jeweils in freier Form oder in Salzform, in eine andere Verbindung der Formel I oder ein Tautomeres davon überführt, ein verfahrensgemäss erhältliches Gemisch von Isomeren auftrennt und das gewünschte Isomere isoliert und/oder eine verfahrensgemäss erhältliche freie Verbindung der Formel I oder ein Tautomeres davon in ein Salz oder ein verfahrensgemäss erhältliches Salz einer Verbindung der Formel I oder eines Tautomeren davon in die freie Verbindung der Formel I oder ein Tautomeres davon oder in ein anderes Salz überführt.

Für vor- und nachstehend aufgeführte Ausgangsmaterialien gilt im Hinblick auf deren Tautomere bzw. Salze das vorstehend für Tautomere bzw. Salze von Verbindungen der Formel I Gesagte in analoger Weise.

Die vor- und nachstehend beschriebenen Umsetzungen werden in an sich bekannter Weise durchgeführt, z. B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter Erwärmen, z. B. in einem Temperaturbereich von etwa -80°C bis zur Siedetemperatur des Reaktionsmediums, vorzugsweise von etwa -20°C bis etwa +150°C, und, falls erforderlich, in einem geschlossenen Gefäss, unter Druck, in einer Inertgasatmosphäre und/oder unter wasserfreien Bedingungen arbeitet. Besonders vorteilhafte Reaktionsbedingungen können den Beispielen entnommen werden.

Die vor- und nachstehend aufgeführten Ausgangsmaterialien, die für die Herstellung der Verbindungen der Formel I und gegebenenfalls ihrer Tautomeren oder einem Salz davon verwendet werden, sind bekannt oder können nach an sich bekannten Methoden, z. B. gemäss den nachstehenden Angaben, hergestellt werden.

Variante a):

Geeignete Basen zur Erleichterung der HZ-Abspaltung sind z. B. Alkalimetall- oder Erdalkalimetall-hydroxide, -hydride, -amide, -alkanolate, -carbonate, -dialkylamide oder -alkylsilylamide, Alkylamine, Alkylendiamine, gegebenenfalls N-alkylierte, gegebenenfalls ungesättigte, Cycloalkylamine, basische Heterocyclen, Ammoniumhydroxide sowie carbocyclische Amine. Beispielhaft seien Natrium-hydroxid, -hydrid, -amid, -methanolat, -carbonat, Kalium-tert.-butanolat, -carbonat, Lithiumdiisopropylamid, Kalium-bis-(trimethylsilyl)-amid, Calciumhydrid, Triethylamin, Triethylendiamin, Cyclohexylamin, N-Cyclohexyl-N,N-dimethyl-amin, N,N-Diethylanilin, Pyridin, 4-(N,N-Dimethylamino)pyridin, N-Methylmorpholin, Benzyl-trimethylammoniumhydroxid sowie 1,5-Diazabicyclo[5.4.0]undec-5-en (DBU) genannt.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethen; Ester, wie Essigsäureethylester; Ether, wie Diethylether,Ethylenglykolmonomethylether, Ethylenglykolmonoethylether, Ethylenglykoldimethylether, tert.-Butylmethylether, Tetrahydrofuran oder Dioxan; Ketone, wie Aceton, Methylethylketon oder Methylisobutylketon; Alkohole, wie Methanol, Ethanol, Propanol, Butanol, Ethylenglykol oder Glycerin; Amide, wie N,N-Dimethylformamid, N,N-Dimethylacetamid, N-Methylpyrrolidon oder Hexamethylphosphorsäuretriamid; Nitrile, wie Acetonitril; und Sulfoxide, wie Dimethylsulfoxid. Auch im Ueberschuss eingesetzte Basen, wie Triethylamin, Pyridin, N-Methylmorpholin oder N,N-Diethylanilin, können als Lösungs- oder Verdünnungsmittel dienen.

Die Umsetzung erfolgt vorteilhaft in einem Temperaturbereich von etwa -80°C bis etwa +120°C, bevorzugt von etwa -20°C bis etwa +50°C.

In einer bevorzugten Ausführungsform der Variante a) wird eine Verbindung der Formel II bei etwa 0°C bis etwa 20°C in einem Ether, vorzugsweise in Tetrahydrofuran, und in Gegenwart eines Alkylamins, vorzugsweise in Gegenwart von Triethylamin, mit einer Verbindung der Formel III, worin Z Halogen, vorzugsweise Chlor, ist, umgesetzt.

Die Verbindungen der Formel II oder eines ihrer Salze und die Verbindungen der Formel III und gegebenenfalls deren Tautomere, beziehungsweise ein Salz davon, sind bekannt oder können in Analogie zu bekannten Verbindungen hergestellt werden.

Variante b):

Geeignete Säuren zur Erleichterung der Kondensation sind beispielsweise diejenigen, die vorstehend als für die Bildung von Säureadditionssalzen mit Verbindungen der Formel I geeignet aufgeführt sind.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien genannt: aromatische, aliphatische und alicyclische Kohlenwasserstoffe und Halogenkohlenwasserstoffe, wie Benzol, Toluol, Xylol, Chlorbenzol, Brombenzol, Petrolether, Hexan, Cyclohexan, Dichlormethan, Trichlormethan, Dichlorethan oder Trichlorethen; Ester, wie Essigsäureethylester; Ether, wie Diethylether,Ethylenglykolmonomethylether, Ethylenglykolmonoethylether, Ethylenglykoldimethylether, tert.-Butylmethylether, Tetrahydrofuran oder Dioxan; Ketone, wie Aceton, Methylethylketon oder Methylisobutylketon; Alkohole, wie Methanol, Ethanol, Propanol, Butanol, Ethylenglykol oder Glycerin; Amide, wie N,N-Dimethylformamid, N,N-Dimethylacetamid, N-Methylpyrrolidon oder Hexamethylphosphorsäuretriamid; Nitrile, wie Acetonitril; Sulfoxide, wie Dimethylsulfoxid; und Säuren, vorzugsweise starke organische Carbonsäuren, wie gegebenenfalls, z. B. durch Halogen, substituierte $C_1$-$C_4$-Alkancarbonsäuren, z. B. Ameisensäure, Essigsäure oder Propionsäure.

Die Umsetzung erfolgt vorteilhaft in einem Temperaturbereich von etwa 0°C bis etwa +180°C, bevorzugt von etwa +20°C bis etwa +130°C, in vielen Fällen bei der Rückflusstemperatur des verwendeten Lösungsmittel.

Die Verbindungen der Formel IV sowie die Verbindungen der Formel V und gegebenenfalls deren Tautomere, sowie deren Salze, sind bekannt oder können in Analogie zu bekannten Verbindungen hergestellt werden.

Variante c):

Geeignete Basen zur Erleichterung der HX-Abspaltung sind beispielsweise die unter der Variante a) aufgeführten Basen.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien die unter der Variante a) aufgeführten Lösungs- oder Verdünnungsmittel genannt. Auch im Ueberschuss eingesetzte Reaktionspartner der Verbindungen der Formel VI [Wasser, $H_2S$, $C_1$-$C_4$-Alkanole, Mercapto-$C_1$-$C_4$-alkane, Verbindungen der Formel $HN(R_6)R_7$ oder Verbindungen der Formel $HN(R_4)R_5$] können als Lösungs- oder Verdünnungsmittel dienen.

Die Umsetzung erfolgt vorteilhaft in einem Temperaturbereich von etwa -80°C bis etwa +120°C, bevorzugt von etwa -10°C bis etwa +80°C.

Die Verbindungen der Formel VI und gegebenenfalls deren Tautomere, jeweils in freier Form oder in Salzform, können in an sich bekannter Weise hergestellt werden, z. B. wie unter der Verfahrensvariante e) beschrieben.

Die als Reaktionspartner der Verbindungen der Formel VI eingesetzten $C_1$-$C_4$-Alkanole, Mercapto-$C_1$-$C_4$-alkane und Verbindungen der Formel $HN(R_4)R_5$ sowie deren Salze sind bekannt oder können in Analogie zu bekannten Verbindungen hergestellt werden.

Variante d):

Geeignete Basen zur Erleichterung der HX-Abspaltung sind beispielsweise die unter der Variante a) aufgeführten Basen.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien die unter der Variante a) aufgeführten Lösungs- oder Verdünnungsmittel genannt. Auch im Ueberschuss eingesetzte Reaktionspartner der Verbindungen der Formel VII [Verbindungen der Formel $HN(R_6)R_7$] können als Lösungs- oder Verdünnungsmittel dienen.

Die Umsetzung erfolgt vorteilhaft in einem Temperaturbereich von etwa -80°C bis etwa +150°C, bevorzugt von etwa 0°C bis etwa +100°C.

In einer bevorzugten Ausführungsform der Variante d) wird eine Verbindung der Formel VII bei etwa 0°C bis etwa +80°C in einem Ester, vorzugsweise in Essigsäureethylester, und in Gegenwart eines Akylamins, vorzugsweise in Gegenwart von Triethylamin, mit einer Verbindung der Formel $HN(R_6)R_7$ umgesetzt.

Die Verbindungen der Formel VII sowie deren Salze und die als Reaktionspartner der Verbindungen der Formel VII eingesetzten Verbindungen der Formel $HN(R_6)R_7$ sowie deren Salze sind bekannt oder können in Analogie zu bekannten Verbindungen hergestellt werden.

Variante e):

Geeignete Basen zur Neutralisation der entstehenden Halogenwasserstoffsäure sind beispielsweise die unter der Variante a) aufgeführten Basen.

Die Reaktionspartner können als solche, d. h. ohne Zusatz eines Lösungs- oder Verdünnungsmittels, z. B. in der Schmelze, miteinander umgesetzt werden. Zumeist ist jedoch der Zusatz eines inerten Lösungs- oder Verdünnungsmittels oder eines Gemisches derselben vorteilhaft. Als Beispiele für solche Lösungs- oder Verdünnungsmittel seien die unter der Variante a) aufgeführten Lösungs- oder Verdünnungsmittel genannt.

Die Umsetzung erfolgt vorteilhaft in einem Temperaturbereich von etwa -80°C bis etwa +180°C, bevorzugt von etwa 0°C bis etwa +100°C.

Die Verbindungen der Formel I, worin $R_3$ für -OH oder -SH steht, und deren Tautomere sowie deren Salze können in an sich bekannter Weise hergestellt werden, z. B. wie unter der Verfahrensvariante a) beschrieben.

Eine verfahrensgemäss oder auf andere Weise erhältliche Verbindung der Formel I oder gegebenenfalls ein Tautomeres davon kann in an sich bekannter Weise in eine andere Verbindung der Formel I überführt werden, indem man einen oder mehrere Substituenten der Ausgangsverbindung der Formel I in üblicher Weise durch (einen) andere(n) erfindungsgemässe(n) Substituenten ersetzt.

Beispielsweise können:

- Hydroxygruppen $R_3$ zu $C_1$-$C_4$-Alkoxygruppen $R_3$ alkyliert werden;
- Mercaptogruppen $R_3$ zu $C_1$-$C_4$-Alkylthiogruppen $R_3$ alkyliert werden; oder
- Aminogruppen $R_3$ zu mono- oder diakylierten Aminogruppen $R_3$ alkyliert werden.

Es ist dabei, je nach Wahl der dafür jeweils geeigneten Reaktionsbedingungen und Ausgangsmaterialien, möglich, in einem Reaktionsschritt nur einen Substituenten durch einen anderen erfindungsgemässen Substituenten zu ersetzen, oder es können in demselben Reaktionschritt mehrere Substituenten durch andere erfindungsgemässe Substituenten ersetzt werden.

Salze von Verbindungen der Formel I können in an sich bekannter Weise hergestellt werden. So erhält man beispielsweise Säureadditionssalze von Verbindungen der Formel I durch Behandeln mit einer geeigneten Säure oder einem geeigneten Ionenaustauscherreagens und Salze mit Basen durch Behandeln mit einer geeigneten Base oder einem geeigneten Ionenaustauscherreagens.

Salze von Verbindungen der Formel I können in üblicher Weise in die freien Verbindungen der Formel I überführt werden, Säureadditionssalze z. B. durch Behandeln mit einem geeigneten basischen Mittel oder einem geeigneten Ionenaustauscherreagens und Salze mit Basen z. B. durch Behandeln mit einer geeigneten Säure oder einem geeigneten Ionenaustauscherreagens.

Salze von Verbindungen der Formel I können in an sich bekannter Weise in andere Salze von Verbindungen der Formel I umgewandelt werden, Säureadditionssalze beispielsweise in andere Säureadditionssalze, z. B. durch Behandeln eines Salzes einer anorganischen Säure, wie eines Hydrochlorids, mit einem geeigneten Metallsalz, wie einem Natrium-, Barium- oder Silbersalz, einer Säure, z. B. mit Silberacetat, in einem geeigneten Lösungsmittel, in welchem ein sich bildendes anorganisches Salz, z.B. Silberchlorid, unlöslich ist und damit aus dem Reaktionsgemisch ausscheidet.

Je nach Verfahrensweise bzw. Reaktionsbedingungen können die Verbindungen der Formel I mit salzbildenden Eigenschaften in freier Form oder in Form von Salzen erhalten werden.

Die Verbindungen der Formel I und gegebenenfalls ihre Tautomeren, jeweils in freier Form oder in Salzform, können in Form eines der möglichen Isomeren oder als Gemisch derselben, z. B., je nach Anzahl, absoluter und relativer Konfiguration von im Molekül auftretenden asymmetrischen Kohlenstoffatomen und/oder je nach Konfiguration von im Molekül auftretenden nichtaromatischen Doppelbindungen, als reine Isomere, wie Antipoden und/oder Diastereomere, oder als Isomerengemische, wie Enantiomerengemische, z.B. Racemate, Diastereomerengemische oder Racematgemische, vorliegen; insbesondere sind darunter auch Syn- und Antiisomere zu verstehen, welche sich aufgrund der vorhandenen C=N-Doppelbindungen ergeben können, wobei gemäss der Zahl dieser Doppelbindungen Gemische von Syn/anti-Isomerenpaaren anfallen können. Die Erfindung betrifft sowohl die reinen Isomeren als auch alle möglichen Isomerengemische und ist vor- und nachstehend jeweils entsprechend zu verstehen, auch wenn stereochemische Einzelheiten nicht in jedem Fall speziell erwähnt werden.

Verfahrensgemäss - je nach Wahl der Ausgangsstoffe und Arbeitsweisen - oder anderweitig erhältliche Diastereomerengemische und Racematgemische von Verbindungen der Formel I und gegebenenfalls ihrer Tautomeren, jeweils in freier Form oder in Salzform, können auf Grund der physikalisch-chemischen Unterschiede der Bestandteile in bekannter Weise in die reinen Diastereomeren oder Racemate aufgetrennt werden, beispielsweise durch fraktionierte Kristallisation, Destillation und/oder Chromatographie.

Entsprechend erhältliche Enantiomerengemische, wie Racemate, lassen sich nach bekannten Methoden in die optischen Antipoden zerlegen, beispielsweise durch Umkristallisation aus einem optisch aktiven Lösungsmittel, durch Chromatographie an chiralen Adsorbentien, z. B. Hochdruckflüssigkeitschromatographie (HPLC) an Acetylcellulose, mit Hilfe von geeigneten Mikroorganismen, durch Spaltung mit spezifischen, immobilisierten Enzymen, über die Bildung von Einschlussverbindungen, z. B. unter Verwendung chiraler Kronenether, wobei nur ein Enantiomeres komplexiert wird, oder durch Überführung in diastereomere Salze, z. B. durch Umsetzung eines basischen Endstoffracemats mit einer optisch aktiven Säure, wie Carbonsäure, z. B. Campher-, Wein- oder Äpfelsäure, oder Sulfonsäure, z. B. Camphersulfonsäure, und Trennung des auf diese Weise erhaltenen Diastereomerengemisches, z. B. auf Grund ihrer verschiedenen Löslichkeiten durch fraktionierte Kristallisation, in die Diastereomeren, aus denen das gewünschte Enantiomere durch Einwirkung geeigneter, z. B. basischer, Mittel freigesetzt werden kann.

Ausser durch Auftrennung entsprechender Isomerengemische können reine Diastereomere bzw. Enantiomere erfindungsgemäss auch durch allgemein bekannte Methoden der diastereoselektiven bzw. enantioselektiven Synthese erhalten werden, z. B. indem man das erfindungsgemässe Verfahren mit Edukten mit entsprechend geeigneter Stereochemie ausführt.

Vorteilhaft isoliert bzw. synthetisiert man jeweils das biologisch wirksamere Isomere, z. B. Enantiomere oder Diastereomere, oder Isomerengemisch, z. B. Enantiomerengemisch oder Diastereomerengemisch, sofern die einzelnen Komponenten unterschiedliche biologische Wirksamkeit besitzen.

Die Verbindungen der Formel I und gegebenenfalls ihre Tautomeren, jeweils in freier Form oder in Salzform, können auch in Form ihrer Hydrate erhalten werden und/oder andere, beispielsweise gegebenenfalls zur Kristallisation von in fester Form vorliegenden Verbindungen verwendete, Lösungsmittel einschliessen.

Die Erfindung betrifft alle diejenigen Ausführungsformen des Verfahrens, nach denen man von einer auf irgendeiner Stufe des Verfahrens als Ausgangs- oder Zwischenprodukt erhältlichen Verbindung ausgeht und alle oder einige der fehlenden Schritte durchführt oder einen Ausgangsstoff in Form eines Derivates bzw. Salzes und/oder seiner Racemate bzw. Antipoden verwendet oder insbesondere unter den Reaktionsbedingungen bildet.

Beim Verfahren der vorliegenden Erfindung werden vorzugsweise solche Ausgangsstoffe und Zwischenprodukte sowie deren Salze verwendet, welche zu den eingangs als besonders wertvoll geschilderten Verbindungen der Formel I bzw. deren Salzen führen.

Die Erfindung betrifft insbesondere die in den Beispielen H1 bis H3 beschriebenen Herstellungsverfahren.

Die erfindungsgemäss für die Herstellung der Verbindungen der Formel I bzw. ihrer Salze verwendeten Ausgangsstoffe und Zwischenprodukte sowie deren Salze, die neu sind, ihre Verwendung und Verfahren zu ihrer Herstellung bilden ebenfalls einen Gegenstand der Erfindung.

Die erfindungsgemässen Verbindungen der Formel I sind bei günstiger Warmblüter-, Fisch- und Pflanzenverträglichkeit wertvolle Wirkstoffe auf dem Gebiet der Schädlingsbekämpfung. Insbesondere wirken die erfindungsgemässen Wirkstoffe gegen Insekten und Spinnentiere, wie sie an Nutz- und Zierpflanzen in der Landwirtschaft und im Gartenbau, insbesondere in Baumwoll-, Gemüse- und Obstpflanzungen, und im Forst vorkommen. Weitere Anwendungsgebiete der erfindungsgemässen Wirkstoffe sind der Vorrats- und Materialschutz sowie im Hygienesektor insbesondere der Schutz von Haus- und Nutztieren. Die Verbindungen der Formel I sind gegen alle oder einzelne Entwicklungsstadien von normal sensiblen, aber auch von resistenten Arten von Schädlingen wirksam. Dabei kann sich ihre Wirkung z. B. in einer Abtötung der Schädlinge, welche unmittelbar oder erst nach einiger Zeit, beispielsweise bei einer Häutung, eintritt, oder in einer verminderten Eiablage und/oder Schlupfrate zeigen.

Zu den oben erwähnten Schädlingen gehören:

aus der Ordnung Lepidoptera zum Beispiel

Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;

aus der Ordnung Coleoptera zum Beispiel

Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp., Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;

aus der Ordnung Orthoptera zum Beispiel

Blatta spp., Blattella spp., Gryllotalpa spp., Leucophaea maderae, Locusta spp., Periplaneta spp. und Schistocerca spp.;

aus der Ordnung Isoptera zum Beispiel

Reticulitermes spp.;

aus der Ordnung Psocoptera zum Beispiel

Liposcelis spp.;
aus der Ordnung Anoplura zum Beispiel
Haematopinus spp., Linognathus spp., Pediculus spp., Pemphigus spp. und Phylloxera spp.;
aus der Ordnung Mallophaga zum Beispiel
Damalinea spp. und Trichodectes spp.;
aus der Ordnung Thysanoptera zum Beispiel
Frankliniella spp., Hercinothrips spp., Taeniothrips spp., Thrips palmi, Thrips tabaci und Scirtothrips aurantii;
aus der Ordnung Heteroptera zum Beispiel
Cimex spp., Distantiella theobroma, Dysdercus spp., Euchistus spp. Eurygaster spp. Leptocorisa spp., Nezara spp., Piesma spp., Rhodnius spp., Sahlbergella singularis, Scotinophara spp. und Triatoma spp.;
aus der Ordnung Homoptera zum Beispiel
Aleurothrixus floccosus, Aleyrodes brassicae, Aonidiella spp., Aphididae, Aphis spp., Aspidiotus spp., Bemisia tabaci, Ceroplaster spp., Chrysomphalus aonidium,
Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp.,
Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp.,
Scaphoideus spp., Schizaphis spp., Sitobion spp., Trialeurodes vaporariorum, Trioza erytreae und Unaspis citri;
aus der Ordnung Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung Siphonaptera zum Beispiel
Ceratophyllus spp. und Xenopsylla cheopis und
aus der Ordnung Thysanura zum Beispiel
Lepisma saccharina.

Insbesondere eignen sich die Verbindungen der Formel I zur Bekämpfung von pflanzenschädigende Insekten der Gattungen Spodoptera, Heliothis, Plutella und Diabrotica.

Die gute pestizide Wirkung der erfindungsgemässen Verbindungen der Formel I entspricht einer Abtötungsrate (Mortalität) von mindestens 50-60 % der erwähnten Schädlinge.

Die Wirkung der erfindungsgemässen Verbindungen und der sie enthaltenden Mittel lässt sich durch Zusatz von anderen Insektiziden wesentlich verbreitern und an gegebene Umstände anpassen. Als Zusätze kommen zum Beispiel Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und können daher beispielsweise zu Emulsionskonzentraten, direkt versprüh- oder verdünnbaren Lösungen, verdünnten Emulsionen, Spritzpulvern, löslichen Pulvern, Stäubemitteln, Granulaten, auch Verkapselungen in polymeren Stoffen in bekannter Weise verarbeitet werden. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, das heisst die den Wirkstoff der Formel I, beziehungsweise Kombinationen dieser Wirkstoffe mit anderen Insektiziden, und gegebenenfalls einen festen oder flüssigen Zusatzstoff enthaltenden Mittel, Zubereitungen oder Zusammensetzungen, werden in bekannter Weise hergestellt, zum Beispiel durch inniges Vermischen und/oder Vermahlen der Wirkstoffe mit Streckmitteln, wie beispielsweise mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Ether und Ester, wie Ethanol, Ethylenglykol, Ethylenglykolmonomethyl- oder -ethylether, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie Bimsstein, Ziegelbruch, Sepiolit oder Bentonit, als nicht sorptive Trägermaterialien Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als obeflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen als auch wasserlösliche synthetische obeflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie die Natrium- oder Kalium-Salze der Oel- oder Stearinsäure, oder von natürlichen Fettsäuregemischen, die beispielsweise aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, beispielsweise das Natrium- oder Calcium-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind zum Beispiel die Natrium-, Calcium- oder Triethanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie zum Beispiel Salze des Phosphorsäureesters eines p-Nonylphenol-(4-14)-Ethylenoxid-Adduktes, in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Ethylenglykolethergruppen und 10 bis 100 Propylenglykolethergruppen enthaltenden Polyethylenoxid-Addukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykol-Einheiten.

Als Beispiele nichtionischer Tenside seien Nonylphenolpolyethoxyethanole, Ricinusölpolyglykolether, Ricinusölthioxilat, Polypropylen-Polyethylenoxid-Addukte, Tributylphenoxypolyethoxyethanol, Polyethylenglykol und Octylphenoxypolyethoxyethanol erwähnt. Ferner kommen auch Fettsäureester von Polyoxyethylensorbitan, wie das Polyoxyethylensorbitan-trioleat in Betracht.

Bei den kationischen Tensiden handelt es sich vor allem um quarternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, beispielsweise das Stearyltrimethylammoniumchlorid oder das Benzyl-di-(2-chlorethyl)-ethylammoniumbromid.

Die pestiziden Zubereitungen enthalten in der Regel 0,1 bis 99%, insbesondere 0,1 bis 95%, Wirkstoff der Formel I oder Kombinationen dieses Wirkstoffs mit andern Insektiziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 1 bis 1000 g Wirkstoff pro Hektar, vorzugsweise 25 bis 500 g/ha.

Insbesondere setzen sich bevorzugte Formulierungen folgendermassen zusammen (% = Gewichtsprozent):

| Emulgierbare Konzentrate: | |
|---|---|
| Wirkstoff: | 1 bis 20 %, vorzugsweise 5 bis 10 % |
| oberflächenaktives Mittel: | 5 bis 30 %, vorzugsweise 10 bis 20 % |
| flüssiges Trägermittel: | 50 bis 94 %, vorzugsweise 70 bis 85 % |

| Stäube: | |
|---|---|
| Wirkstoff: | 0,1 bis 10 %, vorzugsweise 0,1 bis 1 % |
| feste Trägermittel: | 99,9 bis 90 %, vorzugsweise 99,9 bis 99 % |

| Suspension-Konzentrate: | |
|---|---|
| Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

| Benetzbare Pulver: | |
|---|---|
| Wirkstoff: | 0,5 bis 90 %, vorzugsweise 1 bis 80 % |
| oberflächenaktives Mittel: | 0,5 bis 20 %, vorzugsweise 1 bis 15 % |
| feste Trägermaterial: | 5 bis 95 %, vorzugsweise 15 bis 90 % |

| Granulate: | |
|---|---|
| Wirkstoff: | 0,5 bis 30 %, vorzugsweise 3 bis 15 % |
| festes Trägermittel: | 99,5 bis 70 %, vorzugsweise 97 bis 85 % |

Die Mittel können auch weitere Zusätze, wie Stabilisatoren, Entschäumer, Konservierungmittel, Viskositätsregulatoren, Bindemittel, Haftmittel, Dünger oder andere Wirkstoffe, zur Erzielung spezieller Effekte enthalten.

Die folgenden Beispiele dienen der Erläuterung der Erfindung. Sie sollen die Erfindung in keiner Weise einschränken.

Herstellungsbeispiele

Beispiel H1:

1-(4-Chlorphenyl)-4-hydroxy-5-methyl-6-oxa-2,3,5-triaza-1-(4-trifluormethansulfonyloxyphenyl)-hepta-1,3-dien

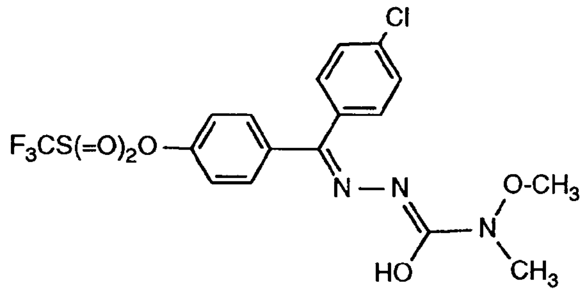

bzw. 1-(4-Chlorphenyl)-5-methyl-6-oxa-4-oxo-2,3,5-triaza-1-(4-trifluormethansulfonyloxyphenyl)-hept-1-en

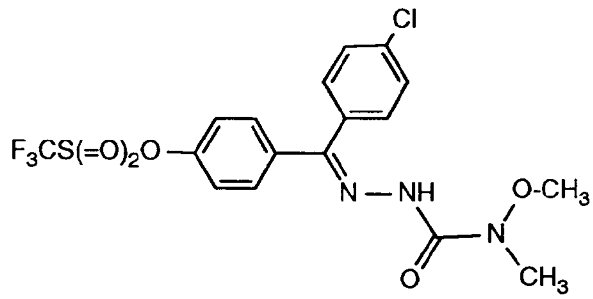

Unter Rühren lässt man zu einer Lösung von 5 g 4-Chlor-4'-trifluormethansulfonyloxybenzophenon-hydrazon in 40 ml Tetrahydrofuran und 2 ml Triethylamin bei 0 bis 10°C 1,8 g N-Methoxy-N-methyl-carbamoylchlorid zutropfen. Der entstandene Niederschlag wird abfiltriert, das Filtrat am Rotationsverdampfer im Vakuum eingedampft und der Rückstand aus Hexan umkristallisiert. Man erhält so die Titelverbindung in Form eines Isomerengemisches, das bei 102 bis 114°C schmilzt (Verbindung Nr.1).

Beispiel H2:

1-(4-Chlorphenyl)-4,5-dimethyl-6-oxa-2,3,5-triaza-1-(4-trifluormethansulfonyloxyphenyl)-hepta-1,3-dien

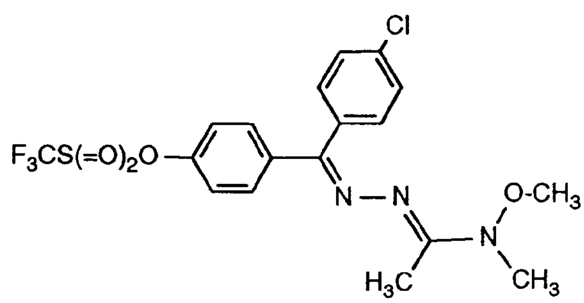

Zu einer Mischung von 32,3 ml Triethylamin, 14,1 g O,N-Dimethylhydroxylamin und 50 ml Essigsäureethylester lässt man bei Raumtemperatur unter Rühren 30,7 g 4-Chlor-1-(4-chlorphenyl)-2,3-diaza-1-(4-trifluormethansulfonyloxyphenyl)-penta-1,3-dien zutropfen. Das Reaktionsgemisch wird 3 Stunden bei 50°C gerührt, filtriert, das Filtrat am Rotationverdampfer im Vakuum eingedampft und der Rückstand säulenchromatographisch [Kieselgel; Essigsäureethylester/Hexan (5 : 95)] gereinigt. Man erhält so die Titelverbindung in Form eines gelblichen Oels mit einem Brechungsindex $n_D^{21}$ von 1,5741 (Verbindung Nr. 25).

Beispiel H3:

4-Chlor-1-(4-chlorphenyl)-5-methyl-6-oxa-2,3,5-triaza-1-(4-trifluormethansulfonyloxyphenyl)-hepta-1,3-dien

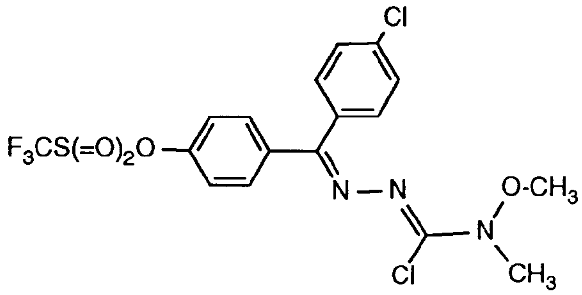

23,3 g 1-(4-Chlorphenyl)-4-hydroxy-5-methyl-6-oxa-2,3,5-triaza-1-(4-trifluormethansulfonyloxyphenyl)-hepta-1,3-dien werden in 200 ml Toluol unter Feuchtigkeitsausschluss mit 11,5 g Phosphorpentachlorid während 30 Minuten verrührt. Man heizt auf 50°C auf und rührt eine Stunde weiter. Man dampft das Lösungsmittel im Vakuum ab, gibt 100 ml Toluol zu und dampft erneut ein. Der ölige Rückstand wird mit 150 ml Hexan vermischt und der entstandene Niederschlag durch Filtration entfernt. Nach Eindampfen des Filtrats erhält man die Titleverbindung als Oel (Verbindung 13).

Beispiel H4:

1-(4-Chlorphenyl)-4-mercapto-5-methyl-6-oxa-2,3,5-triaza-1-(4-trifluormethansulfonyloxyphenyl)-hepta-1,3-dien

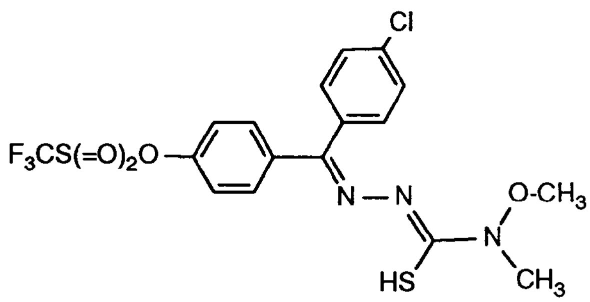

bzw. 1-(4-Chlorphenyl)-5-methyl-6-oxa-2,3,5-triaza-4-thioxo-1-(4-trifluormethansulfonyloxyphenyl)-hept-1-en

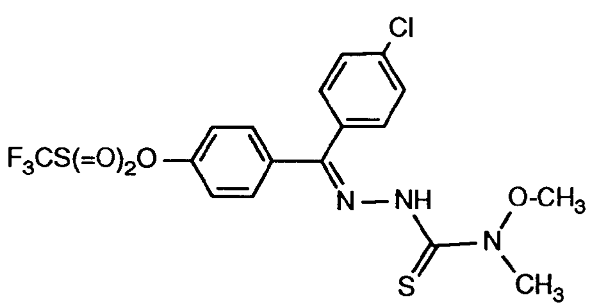

7 g 4-Chlor-1-(4-Chlorphenyl)-5-methyl-6-oxa-2,3,5-triaza-1-(4-trifluormethansulfonyloxyphenyl)-hepta-1,3-dien werden mit 50 ml Tetrahydrofuran, 1,5 g Triethylamin und 0,5 g Schwefelwasserstoff vermischt und 5 Stunden bei Rückflusstemperatur gerührt. Die Reaktionslösung wird eingedampft, der Rückstand in 100 ml Hexan/Essigester-Mischung (10:1) aufgenommen, über Kieselgel filtriert und das Filtrat eingeengt. Man erhält die kristalline Titelverbindung mit einem Schmelzpunkt von 136-137°C (Verbindung Nr.17).

Beispiel H5:

1-(4-Chlorphenyl)-5-methyl-4-methylthio-6-oxa-2,3,5-triaza-1-(4-trifluormethansulfonyloxyphenyl)-hepta-1,3-dien

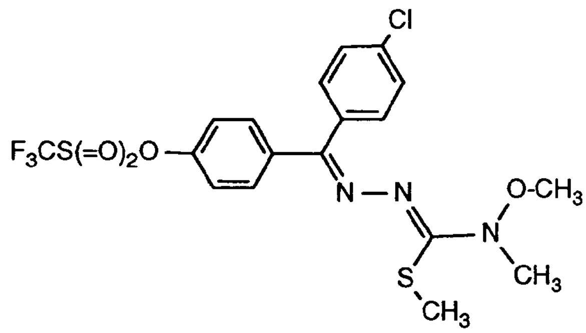

5,8 g 4-Chlor-1-(4-chlorphenyl)-5-methyl-6-oxa-2,3,5-triaza-1-(4-trifluormethansulfonyloxyphenyl)-hepta-1,3-dien werden zusammen mit 50 ml Tetrahydrofuran und 1,2 g Natriumthiomethylat 24 Stunden bei Rückflusstemperatur gerührt. Die Mischung wird eingedampft, der Rückstand in Hexan/Essigsäureethylester (5:1) aufgenommen und an Kieselgel chromatographiert. Man erhält die Titelverbindung als Oel (Verbindung Nr.18).

Beispiel H6:

In analoger Weise wie in den Beispielen H1 bis H5 beschrieben kann man auch die anderen in den Tabellen 1 bis 5 aufgeführten Verbindungen bzw. gegebenenfalls deren Tautomere erhalten. Es handelt sich dabei im allgemeinen um Gemische von Syn- und Antiisomeren. Die mit * bezeichneten Verbindungen sind als reine Syn- bzw. Antiisomere bzw. Isomerenpaare isoliert worden.

Tabelle 1: Verbindungen der Formel

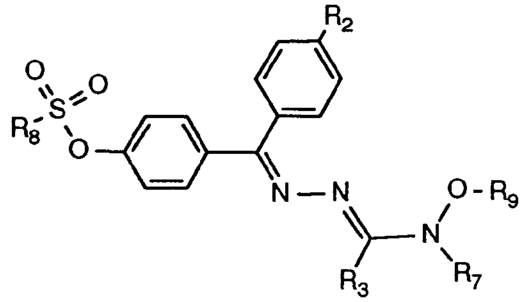

| Verb. Nr. | $R_8$ | $R_2$ | $R_3$ | $R_9$ | $R_7$ | Phys. Daten |
|---|---|---|---|---|---|---|
| 1 | $CF_3$ | Cl | OH | $CH_3$ | $CH_3$ | Smp.102-114°C |
| 2 | $CF_3$ | Cl | OH | $CH_3$ | H | |
| 3 | $CF_3$ | Cl | OH | H | $CH_3$ | |
| 4 | $CF_3$ | Cl | OH | $CH_3$ | $C_2H_5$ | Smp.128-148°C |
| 5 | $CF_3$ | Cl | OH | $C_2H_5$ | $CH_3$ | Smp.115-123°C |
| 6 | $CF_3$ | Cl | OH | $CH_3$ | $CH_2CH=CH_2$ | Smp. 97-102°C |
| 7 | $CF_3$ | Cl | OH | 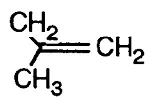 | H | Smp. 50-62°C |
| 8 | $CF_3$ | Cl | OH | $CH_2CH=CHCl$ | H | Smp. 44-47°C |
| 9 | $CF_3$ | Cl | $NH_2$ | $CH_3$ | $CH_3$ | |
| 10 | $CF_3$ | Cl | $N(CH_3)_2$ | $CH_3$ | $CH_3$ | |
| 11 | $CF_3$ | Cl | NHOH | $CH_3$ | $CH_3$ | |
| 12 | $CF_3$ | Cl | $NHCH_3$ | $CH_2CH=CHCH_3$ | $CH_3$ | |
| 13 | $CF_3$ | Cl | Cl | $CH_3$ | $CH_3$ | Oel |
| 14 | $CF_3$ | Cl | $OCH_3$ | $CH_3$ | $CH_3$ | Oel |
| 15 | $CF_3$ | Cl | $OCH_3$ | $CH_3$ | $C_2H_5$ | |
| 16 | $CF_3$ | Cl | $OC_2H_5$ | $CH_3$ | $CH_3$ | |
| 17 | $CF_3$ | Cl | SH | $CH_3$ | $CH_3$ | Smp.136-137°C |
| 18 | $CF_3$ | Cl | $SCH_3$ | $CH_3$ | $CH_3$ | Oel |
| 19 | $CH_3$ | Cl | OH | $CH_3$ | $CH_3$ | Smp.134-146 |
| 20 | $CH_3$ | Cl | OH | H | $CH_3$ | |
| 21 | $CH_3$ | Cl | OH | $CH_3$ | H | |

Tabelle 1 (Fortsetzung)

| Verb. Nr. | $R_8$ | $R_2$ | $R_3$ | $R_9$ | $R_7$ | Phys. Daten |
|---|---|---|---|---|---|---|
| 22 | $CH_3$ | F | OH | $CH_3$ | $CH_3$ | Smp.122-138 |
| 23 | $CF_3$ | F | OH | $CH_3$ | $CH_3$ | Smp.104-110 |
| 24 | $CF_3$ | Br | OH | $CH_3$ | $CH_3$ | |
| 25 | $CF_3$ | Cl | $CH_3$ | $CH_3$ | $CH_3$ | $n_D^{21}$: 1,5741 |
| 26 | $CF_3$ | Cl | H | $CH_3$ | $CH_3$ | $n_D^{25}$:1,5750 |
| 27 | $CF_3$ | Cl | $CH_3$ | H | $CH_3$ | |
| 28 | $CF_3$ | Cl | $CH_3$ | $CH_3$ | H | Smp.121-123 |
| 29 | $CF_3$ | Cl | $CH_3$ | $CH_3$ | $C_2H_5$ | Oel |
| 30 | $CF_3$ | Cl | $CH_3$ | $CH_3$ | $C_3H_7$ | |
| 31 | $CF_3$ | Cl | $CH_3$ | $CH_3$ | $CH_2CH=CH_2$ | |
| 32 | $CF_3$ | Cl | $CH_3$ | H | $C_2H_5$ | |
| 33 | $CF_3$ | Cl | $CH_3$ | H | $CH_2CH=CHCl$ | |
| 34 | $CF_3$ | Cl | H | $CH_3$ | H | |
| 35 | $CF_3$ | Cl | H | $C_2H_5$ | $CH_3$ | |
| 36 | $CF_3$ | Cl | H | H | H | |
| 37 | $CF_3$ | Cl | $CH_3$ | H | H | |
| 38 | $CF_3$ | Cl | H | $CH_2CH=CH_2$ | H | |
| 39 | $CF_3$ | Cl | H | 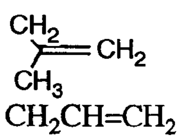 | H | |
| 40 | $CF_3$ | Cl | H | $CH_2CH=CH_2$ | H | |
| 41 | $CF_3$ | Cl | c-$C_3H_5$ | $CH_3$ | $CH_3$ | Harz |
| 42 | $CF_3$ | Cl | $C_2H_5$ | $CH_3$ | $CH_3$ | |
| 43 | $CF_3$ | Cl | c-$C_3H_5$ | $CH_3$ | $C_2H_5$ | |
| 44 | $CF_3$ | Cl | $C_3H_7$ | $CH_3$ | $CH_3$ | |
| 45 | $CF_3$ | Cl | i-$C_3H_7$ | $CH_3$ | $CH_3$ | |
| 46 | $CF_3$ | Cl | $CH_3$ | t-$C_4H_9$ | H | |
| 47 | $CF_3$ | Cl | $CH_3$ | H | t-$C_4H_9$ | |
| 48 | $CF_3$ | Cl | $C_4H_9$ | $CH_3$ | $CH_3$ | |

Tabelle 1 (Fortsetzung)

| Verb. Nr. | $R_8$ | $R_2$ | $R_3$ | $R_9$ | $R_7$ | Phys. Daten |
|---|---|---|---|---|---|---|
| 49 | $CF_3$ | Cl | $CH_2Cl$ | $CH_3$ | $CH_3$ | |
| 50 | $CF_3$ | Cl | $CH_2Br$ | $CH_3$ | $CH_3$ | |
| 51 | $CF_3$ | F | $CH_3$ | $CH_3$ | $CH_3$ | Honig |
| 52 | $CF_3$ | Br | $CH_3$ | $CH_3$ | $CH_3$ | |
| 53 | $CF_3$ | $CF_3$ | $CH_3$ | $CH_3$ | $CH_3$ | |
| 54 | $CH_3$ | Cl | $CH_3$ | $CH_3$ | $CH_3$ | Smp.64-82°C |
| 55 | $CH_2Cl$ | Cl | $CH_3$ | $CH_3$ | $CH_3$ | Oel |
| 56 | $CH_3$ | F | $CH_3$ | $CH_3$ | $CH_3$ | Harz |
| 57 | $CH_2CF_3$ | Cl | $CH_3$ | $CH_3$ | $CH_3$ | |
| 58 | $CH_3$ | Cl | $CH_3$ | H | $CH_3$ | |
| 59 | $CH_3$ | Cl | c-$C_3H_5$ | $CH_3$ | $CH_3$ | |
| 60 | $CF_3$ | Cl | $CH_3$ | 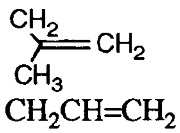 | H | Smp.88-102°C |
| 61 | $CF_3$ | Cl | $CH_3$ | $CH_2CH{=}CH_2$ | H | Smp.86-92°C |
| 62 | $CF_3$ | Cl | OH | $C_2H_5$ | $C_2H_5$ | Smp.130-135°C |
| 63 | $CF_3$ | Cl | $CH_3$ | $CH_2CH{=}CHCl$ | H | Oel |
| 64 | $CF_3$ | F | $CH_3$ | $C_2H_5$ | $CH_3$ | Harz |
| 65 | $CF_3$ | Cl | $CH_3$ | $C_2H_5$ | H | Oel |
| 66 | $CF_3$ | Cl | $CH_3$ | $C_2H_5$ | $CH_3$ | Oel |
| 67* | $CF_3$ | F | $CH_3$ | $CH_3$ | $C_2H_5$ | Smp.84-86°C |
| 68 | $CF_3$ | F | c-$C_3H_5$ | $CH_3$ | $CH_3$ | Harz |
| 69 | $CH_3$ | Cl | $CH_3$ | $C_2H_5$ | $CH_3$ | Smp.72-75°C |
| 70 | $CF_3$ | Cl | $CH_3$ | $C_2H_5$ | $C_2H_5$ | Oel |
| 71 | $CF_3$ | F | $CH_3$ | $CH_2CH{=}CH_2$ | H | Smp.56-60°C |
| 72 | $CF_3$ | F | $CH_3$ | $CH_2CH{=}CHCl$ | H | Harz |
| 73 | $CF_3$ | Cl | OH | $CH_2CH{=}CH_2$ | H | Smp.86-92°C |
| 74 | $CF_3$ | Cl | OH | $C_2H_5$ | H | Smp.80-120°C |
| 75 | $CF_3$ | Br | OH | $CH_3$ | $CH_3$ | |
| 76 | $CF_3$ | Br | $CH_3$ | $CH_3$ | $CH_3$ | Oel |

Tabelle 1 (Fortsetzung)

| Verb. Nr. | $R_8$ | $R_2$ | $R_3$ | $R_9$ | $R_7$ | Phys. Daten |
|---|---|---|---|---|---|---|
| 77 | $CF_3$ | Br | $CH_3$ | $CH_2CH=CH_2$ | $CH_3$ | |
| 78 | $CF_3$ | Br | $CH_3$ | H | $CH_3$ | |
| 79 | $CF_3$ | Br | $CH_3$ | $CH_3$ | H | |

Tabelle 2: Verbindungen der Formel

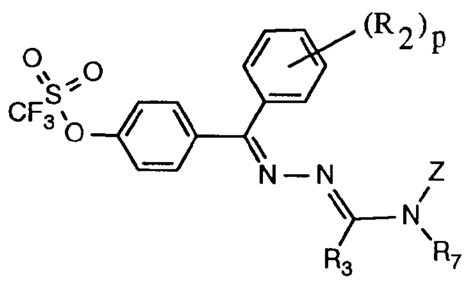

| Verb. Nr. | $R_2$ | $R_3$ | Z | $R_7$ | Phys. Daten |
|---|---|---|---|---|---|
| 80 | 3-Cl | OH | $OCH_3$ | $CH_3$ | |
| 81 | 3-Cl | $CH_3$ | $OCH_3$ | $CH_3$ | Honig |
| 82 | 3-Cl | $CH_3$ | $OCH_2CH=CH_2$ | $CH_3$ | |
| 83 | 3-Cl | $CH_3$ | OH | $CH_3$ | |
| 84 | 3-Cl | $CH_3$ | $OCH_3$ | H | |
| 85 | 2-Cl | OH | $OCH_3$ | $CH_3$ | |
| 86 | 2-Cl | $CH_3$ | $OCH_3$ | $CH_3$ | |
| 87 | 2-Cl | $CH_3$ | $OCH_2CH=CH_2$ | $CH_3$ | |
| 88 | 2-Cl | $CH_3$ | OH | $CH_3$ | |
| 89 | 2-Cl | $CH_3$ | $OCH_3$ | H | |
| 90 | 3,4-$Cl_2$ | OH | $OCH_3$ | $CH_3$ | |
| 91 | 3,4-$Cl_2$ | $CH_3$ | $OCH_3$ | $CH_3$ | Wachs |
| 92 | 3,4-$Cl_2$ | $CH_3$ | $OCH_2CH=CH_2$ | $CH_3$ | |
| 93 | 3,4-$Cl_2$ | $CH_3$ | OH | $CH_3$ | |
| 94 | 3,4-$Cl_2$ | $CH_3$ | $OCH_3$ | H | |
| 95 | 2,4-$Cl_2$ | OH | $OCH_3$ | $CH_3$ | |

Tabelle 2 (Fortsetzung)

| Verb. Nr. | $R_2$ | $R_3$ | Z | $R_7$ | Phys. Daten |
|---|---|---|---|---|---|
| 96 | 2,4-$Cl_2$ | $CH_3$ | $OCH_3$ | $CH_3$ | |
| 97 | 2,4-$Cl_2$ | $CH_3$ | $OCH_2CH{=}CH_2$ | $CH_3$ | |
| 98 | 2,4-$Cl_2$ | $CH_3$ | OH | $CH_3$ | |
| 99 | 2,4-$Cl_2$ | $CH_3$ | $OCH_3$ | H | |
| 100 | 4-Cl | $CH_3$ | $P(O)(OC_2H_5)_2$ | $CH_3$ | Harz |
| 101 | 4-F | $CH_3$ | $P(O)(OC_2H_5)_2$ | i-$C_3H_7$ | Harz |
| 102 | 4-Cl | $CH_3$ | $SO_2CH_3$ | $CH_3$ | Smp. 76-78°C |
| 103 | 4-Cl | $CH_3$ | $SO_2N(CH_3)_2$ | $CH_3$ | Oel |
| 104 | 4-Cl | OH | $NHCON(CH_3)_2$ | H | Smp.126-133°C |
| 105 | 4-Cl | $SCH_3$ | $NH_2$ | H | |
| 106 | 4-Cl | $CH_3$ | $NH_2$ | H | |
| 107 | 4-Cl | $CH_3$ | $N(CH_3)NH_2$ | $CH_3$ | |
| 108 | 3,4-$Cl_2$ | SH | $OCH_3$ | $CH_3$ | |
| 109 | 4-Cl | SH | $NH_2$ | H | Wachs |
| 110 | 2,4-$Cl_2$ | $OCH_2CH_2OCH_3$ | Z = $OCH_3$ | $CH_3$ | |
| 111 | 3-Cl,4-$OCH_3$ | Cl | $OCH_3$ | $CH_2C\equiv CH$ | |
| 112 | $OC_6H_4$-p-Cl | $CH_3$ | $OCH_3$ | H | |
| 113 | 4-Cl | $CH_3$ | $SO_2C_2H_5$ | $CH_3$ | Smp. 91-96°C |

Tabelle 3: Verbindungen der Formel

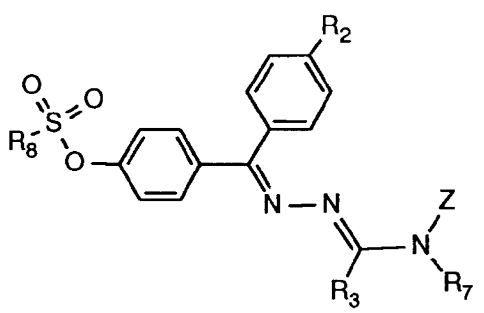

| Verb. Nr. | $R_8$ | $R_2$ | $R_3$ | Z | $R_7$ | Phys. Daten |
|---|---|---|---|---|---|---|
| 114* | $CH_3$ | F | OH | $OCH_3$ | $CH_3$ | Smp.170-173°C |
| 115* | $CH_3$ | Cl | $CH_3$ | $SO_2$-n-$C_4H_9$ | $CH_3$ | Smp. 90-91°C |
| 116* | $CH_3$ | Cl | $CH_3$ | $SO_2$-n-$C_4H_9$ | $CH_3$ | Smp.129-132°C |
| 117 | $CH_3$ | Cl | $CH_3$ | $SO_2CH_3$ | $CH_3$ | Smp.115-142°C |
| 118 | $CH_3$ | Cl | $CH_3$ | $SO_2N(CH_3)_2$ | $CH_3$ | Smp. 73-82°C |
| 119 | $CH_3$ | Cl | $CH_3$ | $SO_2C_2H_5$ | $CH_3$ | Smp.112-143°C |
| 120 | n-$C_3H_7$ | Cl | OH | $NH_2$ | $CH_3$ | |

Tabelle 4: Verbindungen der Formel

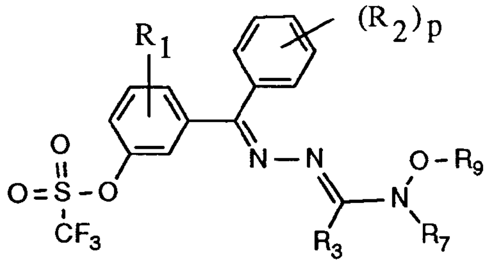

| Verb. Nr. | $R_1$ | $R_2$ | $R_3$ | $R_9$ | $R_7$ | Phys. Daten |
|---|---|---|---|---|---|---|
| 121 | H | 2,4-$Cl_2$ | H | $CH_3$ | $CH_3$ | |
| 122 | 2-Cl | 4-Cl | H | $CH_3$ | $CH_3$ | |
| 123 | H | 4-Cl | Cl | $CH_3$ | $CH_3$ | |
| 124 | 6-Cl | 4-Cl | $CH_3$ | $CH_3$ | $CH_3$ | |
| 125 | H | 2,4-$F_2$ | $CH_3$ | $CH_3$ | $CH_3$ | |
| 126 | H | 3-Cl | H | $CH_3$ | $CH_3$ | |
| 127 | H | 2,4-$Cl_2$ | $CH_3$ | $CH_3$ | $C_2H_5$ | |
| 128 | H | 4-F | OH | $C_2H_5$ | $CH_3$ | |
| 129 | H | 4-Cl | c-$C_3H_5$ | $CH_3$ | $CH_3$ | |

Tabelle 5: Verbindungen der Formel

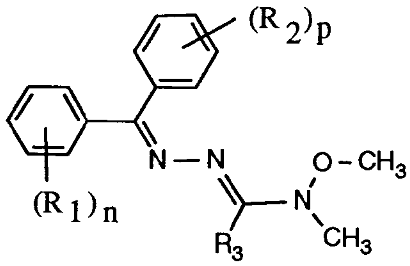

| Verb. Nr. | $R_1$ | $R_2$ | $R_3$ | Phys. Daten |
|---|---|---|---|---|
| 130 | 3,4-($OCF_2O$) | 4-Cl | $CH_3$ | Oel |
| 131 | 4-$OC_6H_5$ | 4-Cl | $CH_3$ | Honig |
| 132 | 4-$OCF_3$ | 4-Cl | $CH_3$ | |
| 133 | 4-$OCF_3$ | 4-F | $CH_3$ | |
| 134 | 2,4-$(OSO_2CF_3)_2$ | 4-Cl | $CH_3$ | |
| 135 | 2,4-$(OSO_2CF_3)_2$ | 4-Cl | Cl | |
| 136 | 2,4-$(OSO_2CF_3)_2$ | 4-Cl | OH | |
| 137 | 2,4-$(OSO_2CF_3)_2$ | 4-Cl | H | |
| 138 | 4-$OCF_2CHF_2$ | 4-Cl | $CH_3$ | |
| 139 | 4-$OCF_2CHFCF_3$ | 4-Cl | $CH_3$ | |
| 140 | 4-$OCF_2CHF_2$ | 4-F | $CH_3$ | |
| 141 | 4-$OCF_2CHFCF_3$ | 4-F | $CH_3$ | |
| 142 | 4-$OCF_2CHFCF_3$ | 4-Cl | OH | Smp. 86-102°C |
| 143 | 4-$OCF_2CHFCF_3$ | 4-F | OH | Smp. 81-88°C |

Formulierungsbeispiele (% = Gewichtsprozent)

| Beispiel F1: Emulsions-Konzentrate | a) | b) | c) |
|---|---|---|---|
| Wirkstoff gemäss Herstellungsbeispielen | 25% | 40 % | 50 % |
| Ca-Dodecylbenzolsulfonat | 5 % | 8 % | 6 % |
| Ricinusölpolyethylenglykolether (36 mol EO) | 5 % | - | - |
| Tributylphenolpolyethylenglykolether (30 mol EO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| Beispiel F2: Lösungen | a) | b) | c) | d) |
|---|---|---|---|---|
| Wirkstoff gemäss Herstellungsbeispielen | 80 % | 10 % | 5 % | 95 % |
| Ethylenglykolmonomethylether | 20 % | - | - | - |
| Polyethylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| Beispiel F3: Granulate | a) | b) |
|---|---|---|
| Wirkstoff gemäss Herstellungsbeispielen | 5 % | 10 % |
| Kaolin | 94 % | - |
| Hochdisperse Kieselsäure | 1 % | - |
| Attapulgit | - | 90 % |

Der Wirkstoff wird in Dichlormethan gelöst, auf den Träger aufgesprüht und das Lösungsmittel anschliessend im Vakuum abgedampft.

| Beispiel F4: Stäubemittel | a) | b) |
|---|---|---|
| Wirkstoff gemäss Herstellungsbeispielen | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| Beispiel F5: Spritzpulver | a) | b) | c) |
|---|---|---|---|
| Wirkstoff gemäss Herstellungsbeispielen | 25 % | 50 % | 75 % |
| Na-Ligninsulfonat | 5 % | 5 % | - |
| Na-Laurylsulfat | 3 % | - | 5 % |
| Na-Diisobutylnaphthalin-sulfonat | - | 6 % | 10 % |
| Octylphenolpolyethylenglykol-ether (7-8 mol EO) | - | 2 % | - |
| Hochdisperse Kieselsäure | 5 % | 10 % | 10 % |
| Kaolin | 62 % | 27 % | - |

Der Wirkstoff wird mit den Zusatzstoffen vermischt und in einer geeigneten Mühle gut vermahlen. Man erhält Spritzpulver, die sich mit Wasser zu Suspensionen jeder gewünschten Konzentration verdünnen lassen.

| Beispiel F6: Emulsions-Konzentrat | |
|---|---|
| Wirkstoff gemäss Herstellungsbeispielen | 10 % |
| Octylphenolpolyethylenglykolether (4-5 mol EO) | 3 % |
| Ca-Dodecylbenzolsulfonat | 3 % |
| Ricinusölpolyglykolether (36 mol EO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| Beispiel F7: Stäubemittel | a) | b) |
|---|---|---|
| Wirkstoff gemäss Herstellungsbeispielen | 5 % | 8 % |
| Talkum | 95 % | - |
| Kaolin | - | 92 % |

Man erhält anwendungsfertige Stäubemittel, indem der Wirkstoff mit dem Träger vermischt und in einer geeigneten Mühle vermahlen wird.

| Beispiel F8: Extruder-Granulat | |
|---|---|
| Wirkstoff gemäss Herstellungsbeispielen | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

| Beispiel F9: Umhüllungs-Granulat | |
|---|---|
| Wirkstoff gemäss Herstellungsbeispielen | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| Beispiel F10: Suspensions-Konzentrat | |
|---|---|
| Wirkstoff gemäss Herstellungsbeispielen | 40 % |
| Ethylenglykol | 10 % |
| Nonylphenolpolyethylenglykolether (15 mol EO) | 6 % |
| Na-Ligninsulfonat | 10 % |
| Carboxymethylcellulose | 1 % |
| 37%-ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele

Beispiel B1: Ovo/larvizide Wirkung auf Heliothis virescens

Auf Baumwolle abgelegte Eier von Heliothis virescens werden mit einer wässrigen Emulsions-Spritzbrühe, die 400 ppm des Wirkstoffes enthält, besprüht. Nach 8 Tagen wird der prozentuale Schlupf der Eier und die Ueberlebensrate der Raupen im Vergleich zu unbehandelten Kontrollansätzen ausgewertet (% Reduktion der Population). Verbindungen der Tabelle 1, 2, 3 und 5 zeigen in diesem Test gute Wirkung.

Beispiel B2: Wirkung gegen Spodoptera littoralis Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des dritten Stadiums von Spodoptera littoralis besiedelt und in einen Plastikbehälter gegeben. 3 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bzw. die prozentuale Reduktion des Frasschadens (% Wirkung) bestimmt.
Verbindungen 1, 4 bis 8, 19, 23, 25, 26, 29, 41, 51, 54, 55, 56, 60, 62 bis 71, 73, 74, 76 100 und 104 zeigen in diesem Test gute Wirkung.

Beispiel B3: Wirkung gegen Diabrotica balteata Larven

Maiskeimlinge werden mit einer wässrigen Emulsions-Spritzbrühe, die 400 ppm des Wirkstoffes enthält, besprüht. Nach dem Antrocknen des Spritzbelages werden die Maiskeimlinge mit 10 Larven des zweiten Stadiums von Diabrotica balteata besiedelt und in einen Plastikbehälter gegeben. 6 Tage später erfolgt die Auswertung. Aus dem Vergleich der Anzahl toter Larven auf den behandelten zu denjenigen auf den unbehandelten Pflanzen wird die prozentuale Reduktion der Population (% Wirkung) bestimmt.
Die Verbindungen 1, 4 bis 8, 13, 19, 22, 23, 25, 26, 28, 29, 41, 51, 54, 55, 56, 61, 62, 64, 65, 66, 69, 70, 71, 73, 74, 100, 102, 104, 113 und 130 zeigen über 80% Wirkung in diesem Test.

Beispiel B4: Wirkung gegen Heliothis virescens Raupen

Junge Sojapflanzen werden mit einer wässrigen Emulsions-Spritzbrühe, die 400 ppm des Wirkstoffes enthält, besprüht. Nach dem Antrocknen des Spritzbelages werden die Sojapflanzen mit 10 Raupen des ersten Stadiums von Heliothis virescens besiedelt und in einen Plastikbehälter gegeben. 6 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bzw. die prozentuale Reduktion des Frasschadens (% Wirkung) bestimmt.
Die Verbindungen 1, 4 bis 8, 13, 17, 19, 22, 23, 25, 26, 29, 41, 51, 54, 55, 56, 61 bis 71, 73, 74, 74, 100, 101, 103, 104, 113 und 130 zeigen über 80% Wirkung in diesem Test.

Beispiel B5: Ovizide Wirkung gegen Heliothis virescens

Auf Filterpapier abgelegte Eier von Heliothis virescens werden für kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).
Die Verbindungen 1, 4 bis 8, 19, 23, 25, 29, 41, 51, 54, 55, 56, 60, 62, 64 bis 70, 73 und 74 zeigen über 80% Wirkung in diesem Test.

Beispiel B6: Wirkung gegen Plutella xylostella Raupen

Junge Kohlpflanzen werden mit einer wässrigen Emulsions- Spritzbrühe, die 400 ppm des Wirkstoffes enthält, besprüht. Nach dem Antrocknen des Spritzbelages werden die Kohlpflanzen mit 10 Raupen des dritten

Stadiums von Plutella xylostella besiedelt und in einen Plastikbehälter gegeben. 3 Tage später erfolgt die Auswertung. Aus dem Vergleich der Anzahl toter Raupen und des Frasschadens auf den behandelten zu denjenigen auf den unbehandelten Pflanzen wird die prozentuale Reduktion der Population bezw. die prozentuale Reduktion des Frasschadens (% Wirkung) bestimmt.

Die Verbindungen 1, 4 bis 8, 19, 23, 25, 26, 29, 41, 51, 54, 55, 56, 60, 62 bis 71, 73, 74 und 76 zeigen über 80% Wirkung gegen Plutella xylostella in diesem Test.

Beispiel B7: Wirkung gegen Blattella germanica

In eine Petri-Schale wird so viel einer 0,1 %igen acetonischen Lösung des Wirkstoffes gegeben, dass die Menge einer Aufwandmenge von 2 g/m2 entspricht. Wenn das Lösungsmittel verdunstet ist, werden 20 Blattella germanica Nymphen (letztes Nymphenstadium) in die so vorbereitete Schale gegeben und 2 Stunden lang der Wirkung der Testsubstanz ausgesetzt. Dann werden die Nymphen mit CO2 narkotisiert, in eine frische Petri-Schale gebracht und im Dunkeln bei 25°C und 50 bis 70 % Luftfeuchtigkeit gehalten. Nach 48 Stunden wird die insektizide Wirkung durch Ermittlung der Abtötungsrate bestimmt.

Verbindungen der Tabellen 1 bis 3 und 5 zeigen gute Wirkung gegen Blattella germanica in diesem Test.

Beispiel B8: Wirkung gegen Schmeissfliegen Lucilia cuprina

Frisch abgelegte Eier der Schmeissfliegenart Lucilia cuprina werden in kleinen Portionen (30 bis 50 Eier) in Reagenzgläser gegeben, in denen zuvor 4 ml Nährmedium mit 1 ml Testlösung, die 16 ppm des zu prüfenden Wirkstoffes enthält, vermischt worden sind. Nach Beimpfung des Kulturmediums werden die Testgläser mit einem Wattestopfen verschlossen und im Brutschrank bei 30°C 4 Tage lang bebrütet. Im unbehandelten Medium entwickeln sich bis zu diesem Zeitpunkt ca. 1 cm lange Larven (Stadium 3). Ist die Substanz aktiv, so sind die Larven zu diesem Zeitpunkt entweder tot oder deutlich zurückgeblieben. Die Auswertung erfolgt nach 96 Stunden.

Die Verbindungen der Tabellen 1 bis 3 und 5 zeigen eine gute Wirkung gegen Lucilia cuprina in diesem Test.

Beispiel B9: Wirkung gegen Ctenocephalides felis

20 bis 25 Floheier werden in eine waagrecht stehend 50-ml-Zellkulturflasche gegeben, in der zuvor 15 g Flohlarven-Nährmedium, welches 100 ppm des zu prüfenden Wirkstoffs enthält, vorgelegt wurde. Die Testflaschen werden in einem Brutschrank bei 26 bis 27°C und 60-70 % Luftfeuchtigkeit bebrütet. Nach 21 Tagen wird die Anwesenheit von adulten Flöhen, nicht geschlüpften Puppen und Larven überprüft.

Die Verbindungen der Tabellen 1 bis 3 und 5 zeigen eine gute Wirkung gegen Ctenocephalides felis in diesem Test.

Beispiel B10: Wirkung gegen Musca domestica

Ein Zuckerwürfel wird mit einer Lösung der Testsubstanz so behandelt, das die Konzentration von Testsubstanz, nach Trocknen über Nacht, im Zucker 250 ppm beträgt. Dieser behandelte Würfel wird mit einem nassen Wattebausch und 10 Adulten Musca domestica eines OP resistenten Stammes auf eine Aluminiumschale gelegt, mit einem Becherglas abgedeckt und bei 25°C inkubiert. Nach 24 Stunden wird die Mortalitätsrate bestimmt.

Die Verbindungen der Tabellen 1 bis 3 und 5 zeigen eine gute Wirkung gegen Musca domestica in diesem Test.

## Patentansprüche

1. Eine Verbindung der Formel

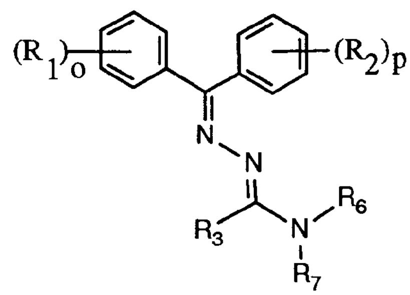

(I),

worin

o und p unabhängig voneinander 0, 1, 2, 3, 4 oder 5, wobei, wenn o grösser als 1 ist, die Reste $R_1$ gleich oder verschieden sind und wobei, wenn p grösser als 1 ist, die Reste $R_2$ gleich oder verschieden sind;

$R_1$ und $R_2$ unabhängig voneinander $C_1$-$C_4$-Alkyl, Halogen-$C_1$-$C_4$-alkyl, $C_1$-$C_4$-Alkoxy, Halogen-$C_1$-$C_4$-alkoxy, $C_1$-$C_4$-Alkylthio, Halogen-$C_1$-$C_4$-alkylthio, Halogen, -OH, Nitro, Cyano, Phenoxy, -O-S(=O)-$R_8$ oder -O-S(=O)$_2$-$R_8$, $C_2$-$C_4$-Alkinyl, durch Phenyl oder Halogen substituiertes $C_2$-$C_4$-Alkinyl, $C_2$-$C_4$-Alkenyl, Halogen-$C_2$-$C_4$-alkenyl, und/oder zwei an benachbarte C-Atome des Phenylrings gebundene Substituenten $R_1$ und/oder zwei an benachbarte C-Atome des Phenylrings gebundene Substituenten $R_2$ unabhängig voneinander gemeinsam -$Y_1$-Z-$Y_2$- sind;

$R_3$ Wasserstoff, Halogen, -OH, -SH, $C_1$-$C_4$-Alkyl, $C_3$-$C_6$-Cycloalkyl, Halogen-$C_1$-$C_4$-alkyl, $C_1$-$C_4$-Alkoxy, $C_1$-$C_4$-Alkoxy-$C_1$-$C_4$-alkyl, $C_1$-$C_4$-Alkoxy-$C_1$-$C_4$-alkoxy, $C_1$-$C_4$-Alkylthio oder -N($R_4$)$R_5$ ist;

$R_4$ und $R_5$ unabhängig voneinander Wasserstoff, $C_1$-$C_4$-Alkyl oder -OH sind;

$R_6$ -$OR_9$, -S(PO)$_n$$R_{10}$, -P(O)$R_{11}$$R_{12}$ oder -$NR_{13}R_{14}$;

$R_7$ Wasserstoff, $C_1$-$C_4$-Alkyl, $C_3$-$C_6$-Cycloalkyl, Halogen-$C_1$-$C_4$-alkyl, $C_1$-$C_4$-Alkoxy-$C_1$-$C_4$-Alkyl, Allyl, $C_1$-$C_3$-Alkylallyl, Halogenallyl oder Propargyl;

$R_8$ $C_1$-$C_8$-Alkyl oder Halogen-$C_1$-$C_8$-alkyl;

$R_9$ Wasserstoff, $C_1$-$C_4$-Alkyl, Halogen-$C_1$-$C_4$-alkyl, $C_1$-$C_4$-Alkoxy-$C_1$-$C_4$-alkyl, $C_3$-$C_6$-Cycloalkyl, $C_2$-$C_6$-Alkenyl, Halogen-$C_2$-$C_6$-alkenyl oder Propargyl;

$R_{10}$ Wasserstoff, $C_1$-$C_4$-Alkyl, Halogen-$C_1$-$C_4$-alkyl, Phenyl oder -$NR_{13}R_{14}$ ist;

$R_{11}$ und $R_{12}$ unabhängig voneinander $C_1$-$C_4$-Alkoxy sind;

$R_{13}$ und $R_{14}$ unabhängig voneinander Wasserstoff, $C_1$-$C_4$-Alkyl, Halogen-$C_1$-$C_4$-alkyl, Phenyl, $C_1$-$C_4$-Alkylamido, $C_1$-$C_4$-Dialkylamido oder -$NH_2$ sind;

n 0, 1 oder 2 ist;

$Y_1$ und $Y_2$ unabhängig voneinander O oder S sind; und

Z Methylen, Eth-1,2-ylen; Halogenmethylen oder Halogeneth-1,2-ylen bedeutet,

Tautomere der Verbindungen der Formel I, sowie Salze der verschiedenen Tautomeren.

2. Eine Verbindung gemäss Anspruch 1 der Formel I, worin
die Reste $R_1$ unabhängig voneinander $C_1$-$C_4$-Alkyl, Halogen-$C_1$-$C_4$-alkyl, $C_1$-$C_4$-Alkoxy, Halogen-$C_1$-$C_4$-alkoxy, Halogen, -O-S(=O)$_2$-$C_1$-$C_4$-Alkyl oder
-O-S(=O)$_2$-Halogen-$C_1$-$C_4$-alkyl, Phenoxy oder zwei Reste $R_1$ gemeinsam -$OCF_2$-O- sind, oder gegebenenfalls Tautomere davon.

3. Eine Verbindung gemäss Anspruch 2 der Formel I, worin
die Reste $R_1$ unabhängig voneinander Halogen-$C_1$-$C_4$-alkyl, Halogen-$C_1$-$C_4$-alkoxy, Halogen, -O-S(=O)$_2$-$C_1$-$C_2$-Alkyl oder -O-S(=O)$_2$-Halogen-$C_1$-$C_2$-alkyl sind, oder gegebenenfalls Tautomere davon.

4. Eine Verbindung gemäss Anspruch 1 der Formel I, worin
die Reste $R_2$ unabhängig voneinander $C_1$-$C_4$-Alkyl, Halogen-$C_1$-$C_4$-alkyl, $C_1$-$C_4$-Alkoxy, Halogen-$C_1$-$C_4$-alkoxy, Halogen, -O-S(=O)$_2$-$C_1$-$C_2$-Alkyl oder -O-S(=O)$_2$-Halogen-$C_1$-$C_2$-alkyl sind, oder gegebenenfalls Tautomere davon.

5. Eine Verbindung gemäss Anspruch 1 der Formel I, worin
$R_3$ Wasserstoff, Halogen, -OH, -SH, $C_1$-$C_4$-Alkyl, $C_3$-$C_6$-Cycloalkyl, Halogen-$C_1$-$C_4$-alkyl, $C_1$-$C_4$-Alkoxy, $C_1$-$C_4$-Alkoxy-$C_1$-$C_4$-alkoxy, $C_1$-$C_4$-Alkylthio, -$NH_2$, -NH($C_1$-$C_2$-Alkyl), -N($C_1$-$C_2$-Alkyl)$_2$ oder -NHOH ist, , oder gegebenenfalls Tautomere davon.

6. Eine Verbindung gemäss Anspruch 1 der Formel I, worin

$R_6$ -$OR_9$ und $R_9$ Wasserstoff, $C_1$-$C_4$-Alkyl, $C_2$-$C_6$-Alkenyl oder Halogen-$C_2$-$C_6$-alkenyl bedeutet, , oder gegebenenfalls Tautomere davon.

7. Eine Verbindung gemäss Anspruch 1 der Formel I, worin
$R_6$ -$SO_2$-$C_1$-$C_4$-Alkyl, -$SO_2NH(CH_3)$, -$P(O)(OC_2H_5)_2$, -$NH_2$, -$N(CH_3)NH_2$ oder -$NHCON(CH_3)_2$ bedeutet, oder gegebenenfalls Tautomere davon.

8. Eine Verbindung gemäss Anspruch 1 der Formel I, worin
$R_7$ Wasserstoff, $C_1$-$C_2$-Alkyl, $C_1$-$C_2$-Alkoxy-$C_1$-$C_2$-alkyl, Allyl, 2-Methylallyl, Halogenallyl oder Propargyl bedeutet, oder gegebenenfalls Tautomere davon.

9. Eine Verbindung gemäss Anspruch 1 der Formel I, worin
$R_1$ -O-$S(=O)_2$-$C_1$-$C_2$-Alkyl, -O-$S(=O)_2$-Halogen-$C_1$-$C_2$-alkyl oder $C_1$-$C_3$-Fluoralkoxy; $R_2$ Fluor oder Chlor;
$R_3$ Wasserstoff, -OH, -SH, $C_1$-$C_2$-Alkyl, Cyclopropyl, $C_1$-$C_2$-Alkoxy, $C_1$-$C_2$-Alkoxy-$C_1$-$C_2$-alkoxy, $C_1$-$C_2$-Alkylthio, -$NH_2$, -$NH(CH_3)$, -$N(CH_3)_2$ oder -NHOH;
$R_6$ -$OR_9$, -$SO_2$-$C_1$-$C_4$-Alkyl, -$SO_2NH(CH_3)$, -$P(O)(OC_2H_5)_2$, -$NH_2$, -$N(CH_3)NH_2$ oder -$NHCON(CH_3)_2$;
$R_7$ Wasserstoff, Methyl oder Ethyl; und
$R_9$ Wasserstoff, $C_1$-$C_2$-Alkyl, $C_2$-$C_3$-Alkenyl oder Halogen-$C_2$-$C_3$-alkenyl und
o und p 1 oder 2 bedeuten, oder gegebenenfalls Tautomere davon.

10. Eine Verbindung gemäss Anspruch 9 der Formel I, worin
$R_1$ -O-$S(=O)_2$-$CH_3$, -O-$S(=O)_2$-$CF_3$ oder -$OCF_2CHFCF_3$;
$R_2$ Chlor;
$R_3$ Wasserstoff, -OH, -SH, $C_1$-$C_2$-Alkyl, Cyclopropyl, $C_1$-$C_2$-Alkoxy, $C_1$-$C_2$-Alkoxy-$C_1$-$C_2$-alkoxy, $C_1$-$C_2$-Alkylthio, -$NH_2$, -$NH(CH_3)$, -$N(CH_3)_2$ oder -NHOH;
$R_6$ -$OR_9$, -$SO_2$-$C_1$-$C_4$-Alkyl, -$SO_2NH(CH_3)$, -$P(O)(OC_2H_5)_2$, -$NH_2$, -$N(CH_3)NH_2$ oder -$NHCON(CH_3)_2$;
$R_7$ Wasserstoff, Methyl oder Ethyl; und
$R_9$ Wasserstoff, $C_1$-$C_2$-Alkyl, $C_2$-$C_3$-Alkenyl oder Halogen-$C_2$-$C_3$-alkenyl und
o und p 1 oder 2 bedeuten, oder gegebenenfalls Tautomere davon.

11. Eine Verbindung gemäss Anspruch 10 der Formel I, worin
$R_1$ in 4-Stellung gebundenes -O-$S(=O)_2$-$CF_3$;
$R_2$ in 4-Stellung gebundenes Chlor;
$R_3$ Wasserstoff, -OH, -SH, $C_1$-$C_2$-Alkyl, Cyclopropyl, $C_1$-$C_2$-Alkoxy, $C_1$-$C_2$-Alkoxy-$C_1$-$C_2$-alkoxy, $C_1$-$C_2$-Alkylthio, -$NH_2$, -$NH(CH_3)$, -$N(CH_3)_2$ oder -NHOH;
$R_6$ -$OR_9$, -$SO_2$-$C_1$-$C_4$-Alkyl, -$SO_2NH(CH_3)$, -$P(O)(OC_2H_5)_2$, -$NH_2$, -$N(CH_3)NH_2$ oder -$NHCON(CH_3)_2$;
$R_7$ Wasserstoff, Methyl oder Ethyl; und
$R_9$ Wasserstoff, $C_1$-$C_2$-Alkyl, $C_2$-$C_3$-Alkenyl oder Halogen-$C_2$-$C_3$-alkenyl und
o und p 1 bedeuten, oder gegebenenfalls Tautomere davon.

12. Eine Verbindung gemäss Anspruch 1 der Formel I, ausgewählt aus der Gruppe von Verbindungen bestehend aus
(a) 1-(4-Chlorphenyl)-4-hydroxy-5-methyl-6-oxa-2,3,5-triaza-1-(4-trifluormethansulfonyloxyphenyl)-hepta-1,3-dien,
bzw. 1-(4-Chlorphenyl)-5-methyl-6-oxa-4-oxo-2,3,5-triaza-1-(4-trifluormethansulfonyloxyphenyl)-hept-1-en;
(b) 1-(4-Chlorphenyl)-4,5-dimethyl-6-oxa-2,3,5-triaza-1-(4-trifluormethansulfonyloxyphenyl)-hepta-1,3-dien;
(c) 1-(4-Chlorphenyl)-4-hydroxy-5-methyl-6-oxa-2,3,5-triaza-1-(4-methansulfonyloxyphenyl)-hepta-1,3-dien,
bzw. 1-(4-Chlorphenyl)-5-methyl-6-oxa-4-oxo-2,3,5-triaza-1-(4-methansulfonyloxyphenyl)-hept-1-en;
(d) 1-(4-Fluorphenyl)-4-hydroxy-5-methyl-6-oxa-2,3,5-triaza-1-(4-trifluormethansulfonyloxyphenyl)-hepta-1,3-dien,
bzw. 1-(4-Fluorphenyl)-5-methyl-6-oxa-4-oxo-2,3,5-triaza-1-(4-trifluormethansulfonyloxyphenyl)-hept-1-en;
(e) 1-(4-Chlorphenyl)-4,5-dimethyl-6-oxa-2,3,5-triaza-1-(4-methansulfonyloxyphenyl)-hepta-1,3-dien;
(f) 1-(4-Chlorphenyl)-4-cyclopropyl-5-methyl-6-oxa-2,3,5-triaza-1-(4-trifluormethansulfonyloxyphenyl)-hepta-1,3-dien;
(g) 1-(4-Fluorphenyl)-4,5-dimethyl-6-oxa-2,3,5-triaza-1-(4-trifluormethansulfonyloxyphenyl)-hepta-1,3-dien;
(h) 1-(4-Chlorphenyl)-4-cyclopropyl-5-ethyl-6-oxa-2,3,5-triaza-1-(4-trifluormethansulfonyloxyphenyl)-hepta-1,3-dien;
(i) 1-(4-Chlorphenyl)-4,5-dimethyl-6-oxa-2,3,5-triaza-1-(4-trifluormethansulfonyloxyphenyl)-octa-1,3-dien;
(j) 1-(4-Chlorphenyl)-5-ethyl-4-methyl-6-oxa-2,3,5-triaza-1-(4-trifluormethansulfonyloxyphenyl)-hepta-1,3-dien, und
(k) 1-(4-Chlorphenyl)-4-hydroxy-5-methyl-6-oxa-2,3,5-triaza-1-(4-trifluormethansulfonyloxyphehyl)-nona-1,3,8-trien, bzw. 1-(4-Chlorphenyl)-5-methyl-6-oxa-4-oxo-2,3,5-triaza-1-(4-trifluormethansulfonyloxyphenyl)-nona-1,8-dien.

13. Verfahren zur Herstellung einer Verbindung gemäss Anspruch 1 der Formel I oder gegebenenfalls eines Tautomeren davon, jeweils in freier Form oder in Salzform, dadurch gekennzeichnet, dass man
a) zur Herstellung einer Verbindung der Formel I, worin $R_3$ für -OH, -SH, -$NHR_4$ oder -$NHR_5$ steht, eine Verbindung der Formel

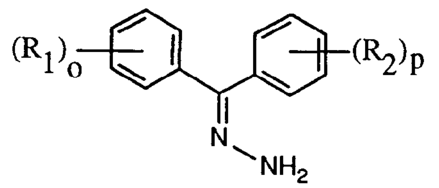

(II)

oder ein Salz davon, vorzugsweise in Gegenwart einer Base, mit einer Verbindung der Formel

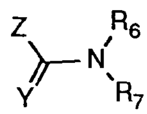

(III),

worin Y für O, S, -$NR_4$ oder -$NR_5$ steht und Z Halogen, $C_1$-$C_8$-Alkoxy, $C_1$-$C_8$-Alkylthio, $C_1$-$C_8$-Alkanoyloxy, $C_1$-$C_8$-Alkansulfonyloxy, Halogen-$C_1$-$C_8$-alkansulfonyloxy, Benzolsulfonyloxy oder Toluolsulfonyloxy bedeutet, oder einem Salz und/oder gegebenenfalls einem Tautomeren davon umsetzt oder
b) eine Verbindung der Formel

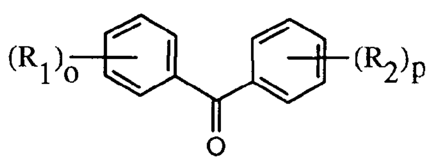

(IV),

mit einer Verbindung der Formel

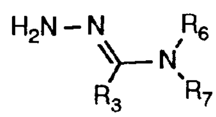

(V)

oder einem Salz und/oder gegebenenfalls einem Tautomeren davon umsetzt oder
c) zur Herstellung einer Verbindung der Formel I, worin $R_3$ -OH, -SH, $C_1$-$C_4$-Alkoxy, $C_1$-$C_4$-Alkylthio oder -$N(R_4)R_5$ ist, eine Verbindung der Formel

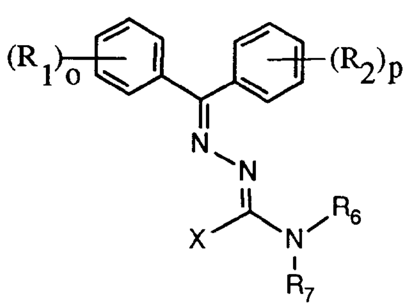

(VI),

worin X Halogen bedeutet, oder ein Salz und/oder gegebenenfalls ein Tautomeres davon, mit Wasser, $H_2S$, einem $C_1$-$C_4$-Alkanol, einem $C_1$-$C_4$-Alkoxy-$C_1$-$C_4$-alkohol, einem Mercapto-$C_1$-$C_4$-alkan oder einer Verbindung der Formel $HN(R_4)R_5$ oder einem Salz davon umsetzt, oder

d) zur Herstellung einer Verbindung der Formel I, worin $R_3$ Wasserstoff, $C_1$-$C_4$-Alkyl, Halogen-$C_1$-$C_4$-Alkyl oder $C_3$-$C_6$-Cycloalkyl bedeutet, eine Verbindung der Formel

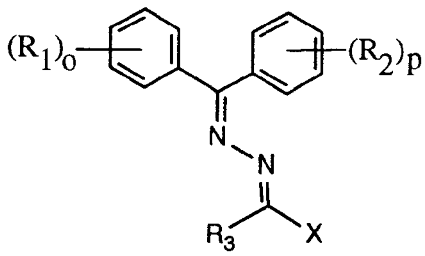

(VII),

worin X Halogen bedeutet, oder ein Salz davon mit einer Verbindung der Formel $HN(R_6)R_7$ oder einem Salz davon umsetzt, oder

e) zur Herstellung einer Verbindung der Formel I, worin $R_3$ Halogen bedeutet, eine Verbindung der Formel I, worin $R_3$ für -OH oder -SH steht, oder ein Salz und/oder gegebenenfalls ein Tautomeres davon mit einem Halogenierungsmittel umsetzt,

und jeweils, wenn erwünscht, eine verfahrensgemäss oder auf andere Weise erhältliche Verbindung der Formel I oder ein Tautomeres davon, jeweils in freier Form oder in Salzform, in eine andere Verbindung der Formel I oder ein Tautomeres davon überführt, ein verfahrensgemäss erhältliches Gemisch von Isomeren auftrennt und das gewünschte Isomere isoliert und/oder eine verfahrensgemäss erhältliche freie Verbindung der Formel I oder ein Tautomeres davon in ein Salz oder ein verfahrensgemäss erhältliches Salz einer Verbindung der Formel I oder eines Tautomeren davon in die freie Verbindung der Formel I oder ein Tautomeres davon oder in ein anderes Salz überführt.

**14.** Mittel zum Schutz gegen den Befall durch Schädlinge, dadurch gekennzeichnet, dass es mindestens eine Verbindung der Formel I gemäss Anspruch 1 oder gegebenenfalls ein Tautomeres davon, jeweils in freier Form oder in agrochemisch verwendbarer Salzform, als Wirkstoff und mindestens einen Hilfsstoff enthält.

**15.** Mittel gemäss Anspruch 14 zum Schutz gegen den Befall durch Insekten und/oder Spinnentiere.

**16.** Verfahren zur Herstellung eines Mittels gemäss Anspruch 14, dadurch gekennzeichnet, dass man den Wirkstoff mit dem (den) Hilfsstoff(en) innig vermischt.

**17.** Verwendung einer Verbindung der Formel I gemäss Anspruch 1 oder gegebenenfalls eines Tautomeren davon, jeweils in freier Form oder in agrochemisch verwendbarer Salzform, oder eines Mittels gemäss Anspruch 14 zum Schutz gegen den Befall durch Schädlinge.

**18.** Verwendung gemäss Anspruch 17 zum Schutz gegen den Befall durch Insekten und/oder Spinnentiere.

**19.** Verfahren zum Schutz gegen den Befall durch Schädlinge, dadurch gekennzeichnet, dass man eine Verbindung der Formel I gemäss Anspruch 1 oder gegebenenfalls ein Tautomeres davon, jeweils in freier Form oder in agrochemisch verwendbarer Salzform, oder ein Mittel gemäss Anspruch 14 auf die Schädlinge und/oder ihren Lebensraum appliziert.

**20.** Verfahren gemäss Anspruch 19 zum Schutz gegen den Befall durch Insekten und/oder Spinnentiere.

Europäisches Patentamt

# EUROPÄISCHER RECHERCHENBERICHT

Nummer der Anmeldung: EP 93810245.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.⁵) |
|---|---|---|---|
| A | WO - A1 - 92/06 076 (E.I. DU PONT DE NEMOURS) * Ansprüche 1,11,12 * -- | 1-3,5, 14,17, 19 | C 07 C 309/65<br>C 07 C 281/14<br>C 07 C 323/48<br>C 07 C 337/08<br>A 01 N 33/26<br>A 01 N 41/04<br>A 01 N 47/34<br>A 01 N 47/42 |
| A | EP - A1 - 0 355 832 (SUMITOMO CHEMICAL) * Ansprüche 1-8 * -- | 1,13, 14,17, 19 | |
| A | EP - A1 - 0 034 010 (GULF OIL) * Ansprüche 1,17-19 * -- | 1,13, 14,17, 19 | |
| A | EP - A1 - 0 026 040 (THE BOOTS COMPANY) * Zusammenfassung * -- | 1,17 | |
| A | US - A - 4 344 893 (LEONARD G. COPPING et al.) * Anspruch 1 * -- | 1 | |
| A | DE - A - 2 044 834 (FARBENFABRIKEN BAYER) * Ansprüche 1,2 * -- | 1,13 | **RECHERCHIERTE SACHGEBIETE (Int. Cl.⁵)** |
| A | US - A - 3 753 680 (HARRY TILLES) * Zusammenfassung * ---- | 1,17 | C 07 C 251/00<br>C 07 C 257/00<br>C 07 C 281/00<br>C 07 C 309/00<br>C 07 C 323/00<br>C 07 C 337/00 |

Der vorliegende Recherchenbericht wurde für alle Patentansprüche erstellt.

| Recherchenort | Abschlußdatum der Recherche | Prüfer |
|---|---|---|
| WIEN | 05-07-1993 | REIF |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82